# EUROPEAN PATENT SPECIFICATION

(11) **EP 1 348 034 B1**
(45) Date of publication and mention of the grant of the patent: **20.07.2016**
(21) Application number: 01997037.5
(22) Date of filing: 15.11.2001
(51) Int. Cl.: C12Q 1/68

(54) **OLIGONUCLEOTIDE IDENTIFIERS**
OLIGONUKLEOTIDIDENTIFIKATOREN
IDENTIFIEURS D'OLIGONUCLEOTIDES

(30) Priority: 15.11.2000 US 248863 P; 22.11.2000 US 252650 P; 15.01.2001 GB 0101054; 19.03.2001 US 276995 P; 29.06.2001 US 302231 P; 03.10.2001 US 326937 P; 03.10.2001 US 327089 P
(43) Date of publication of application: 01.10.2003
(60) Divisional application: 10184188.0
(73) Proprietor: Minerva Biotechnologies Corporation, Waltham, MA 02451 (US)
(72) Inventor: BAMDAD, Cynthia, Carol, Newton, MA 02458 (US); BAMBAD, R., Shoshana, Newton, MA 02458 (US)
(74) Representative: Cowley, Catherine Mary
(86) International application number: PCT/US2001/045845
(87) International publication number: WO 2002/061129

(56) References cited:
- WO-A-00/11446
- WO-A-96/12014
- WO-A-98/22624
- WO-A2-00/43783
- WO-A2-00/43791
- US-A- 5 922 617
- US-A- 6 087 103
- NEEDELS M C ET AL: "GENERATION AND SCREENING OF AN OLIGONUCLEOTIDE-ENCODED SYNTHETIC PEPTIDE LIBRARY" PROCEEDINGS OF THE NATIONAL ACADEMY OF SCIENCES OF USA, NATIONAL ACADEMY OF SCIENCE. WASHINGTON, US, vol. 90, 1 November 1993 (1993-11-01), pages 10700-10704, XP000652260 ISSN: 0027-8424
- DONG KEUN HAN ET AL: "IN VIVO CANINE STUDIES OF A SINKHOLE VALVE AND VASCULAR GRAFT COATED WITH BIOCOMPATIBLE PU-PEO-SO3" ASAIO JOURNAL, J.B.LIPPINCOTT CO.,HAGERSTOWN,MD, US, vol. 39, no. 3, 1 July 1993 (1993-07-01), pages M537-M541, XP000412623 ISSN: 1058-2916
- SIGAL G B ET AL: "SELF-ASSEMBLED MONOLAYER FOR THE BINDING AND STUDY OF HISTIDINE-TAGGED PROTEINS BY SURFACE PLASMON RESONANCE" ANALYTICAL CHEMISTRY, AMERICAN CHEMICAL SOCIETY. COLUMBUS, US, vol. 68, no. 3, 1996, pages 490-497, XP000916112 ISSN: 0003-2700
- LETSINGER R L ET AL: "Use of a steroid cyclic disulfide anchor in constructing gold nanoparticle- oligonucleotide conjugates", BIOCONJUGATE CHEMISTRY, ACS, WASHINGTON, DC, US, vol. 11, no. 2, 1 March 2000 (2000-03-01), pages 289-291, XP002205939, ISSN: 1043-1802, DOI: 10.1021/BC990152N
- M. Junghans: "Antisense delivery using protamine-oligonucleotide particles", Nucleic Acids Research, vol. 28, no. 10, 15 May 2000 (2000-05-15), pages 45e-45, XP055044835, ISSN: 0305-1048, DOI: 10.1093/nar/28.10.e45
- LANGER K ET AL: "Preparation of avidin-labeled protein nanoparticles as carriers for biotinylated peptide nucleic acid", EUROPEAN JOURNAL OF PHARMACEUTICS AND BIOPHARMACEUTICS, ELSEVIER SCIENCE PUBLISHERS B.V., AMSTERDAM, NL, vol. 49, no. 3, 2 May 2000 (2000-05-02), pages 303-307, XP004257171, ISSN: 0939-6411, DOI: 10.1016/S0939-6411(00)00068-0
- C Coester ET AL: "Preparation of avidin-labelled gelatin nanoparticles as carriers for biotinylated peptide nucleic acid (PNA)", International Journal of Pharmaceutics, vol. 196, no. 2, 1 March 2000 (2000-03-01) , pages 147-149, XP055044836, ISSN: 0378-5173, DOI: 10.1016/S0378-5173(99)00409-3
- ANDREW TATON T ET AL: "Scanometric DNA Array Detection with Nanoparticle Probes", SCIENCE, AMERICAN ASSOCIATION FOR THE ADVANCEMENT OF SCIENCE, WASHINGTON, DC; US, vol. 289, 8 August 2000 (2000-08-08), pages 1757-1760, XP002158394, ISSN: 0036-8075, DOI: 10.1126/SCIENCE.289.5485.1757
- L. Ghitescu ET AL: "Immunolabeling efficiency of protein A-gold complexes.", Journal of Histochemistry & Cytochemistry, vol. 38, no. 11, 1 November 1990 (1990-11-01), pages 1523-1530, XP055044840, ISSN: 0022-1554, DOI: 10.1177/38.11.2212613
- T. M. Winger ET AL: "A Convenient Route to Thiol Terminated Peptides for Conjugation and Surface Functionalization Strategies", Bioconjugate Chemistry, vol. 6, no. 3, 1 May 1995 (1995-05-01), pages 323-326, XP055045186, ISSN: 1043-1802, DOI: 10.1021/bc00033a015
- DEMERS L M ET AL: "A FLUORESCENCE-BASED METHOD FOR DETERMINING THE SURFACE COVERAGE AND HYBRIDIZATION EFFICIENCY OF THIOL-CAPPED OLIGONUCLEOTIDES BOUND TO GOLD THIN FILMS AND NANOPARTICLES", ANALYTICAL CHEMISTRY, AMERICAN CHEMICAL SOCIETY, US, vol. 72, no. 22, 15 November 2000 (2000-11-15), pages 5535-5541, XP001204824, ISSN: 0003-2700, DOI: 10.1021/AC0006627
- Harrell Sellers ET AL: "Structure and binding of alkanethiolates on gold and silver surfaces: implications for self-assembled monolayers", Journal of the American Chemical Society, vol. 115, no. 21, 1 October 1993 (1993-10-01), pages 9389-9401, XP055123187, ISSN: 0002-7863, DOI: 10.1021/ja00074a004

## Description

### Field of the Invention

This description relates generally to methods, assays, and components for the rapid, high-throughput, specific and sensitive detection and analysis of biomolecular and chemical interactions, and more particularly to identifiers for identification of participants in these and other assays.

### Background of the Invention

International Patent Application No. PCT/US00/01504, published July 27, 2000 as WO 00/43783 describes a variety of assays involving colloids.

International Patent Application No. PCT/US00/01997, filed January 25, 2000 and U.S. Patent Application Serial No. 09/631,818, filed August 3, 2000 describe methods, assays, and components for analyzing species associated with disease and for screening of candidate drugs for treatment of disease. Assays involving colloid/colloid interaction are described in detail.

*In vitro* techniques that currently exist for studying protein-protein interactions include co-immunoprecipitation, co-fractionation by chromatography, cross-linking, sandwich assays and surface plasmon resonance. A disadvantage of these techniques is that putative binding partners must be sequentially tested which greatly limits the number of potential interacting proteins that can be tested. There are two reasons why experiments have to be performed sequentially. The first is a signaling problem. In a typical binding assay, a single type of signal is produced when binding occurs. Putative binding partners must therefore be kept isolated, then tested sequentially for pair-wise interactions. The second reason for sequential experiments is a bookkeeping problem. Since it is very difficult to identify proteins, especially when at low concentrations, it is necessary to keep track of isolated and purified species, then test for binding in pair-wise fashion.

*In vivo,* cell-based binding assays such as the yeast two hybrid system and yeast mating system provide a major advantage over existing *in vitro* methods in that once a positive protein-protein interaction has been detected, the host cell, which provides the signal, contains ample DNA that codes for the protein(s) under study. As those skilled in the art appreciate, it is far easier to sequence DNA that proteins or peptides. Additionally, DNA at low concentration can be enzymatically amplified prior to sequencing whereas proteins cannot. This eliminates the need for tracking individual aliquots of purified proteins and facilitates high throughput screening to detect protein-protein interactions.

There are, however disadvantages of *in vivo* protein detection systems. *In vivo* assays suffer from false positives and negatives because of the inherent redundancies of the biological processes upon which the assays are based. For example, the yeast two-hybrid system is based on a mechanism of transcriptional activation. Another disadvantage of the system is that it can only detect interactions between cell-derived species. Therefore, interactions between proteins and chemical species, such as drug candidates or chemical recognition elements cannot be detected using this method.

While a wide variety of biological and chemical assay techniques are known, assays with enhanced multiplexing capability that do not sacrifice accuracy in detection would be advantageous. Therefore, an *in vitro* binding assay in which genetic material that codes for expressed proteins or chemical species is available, and can be correlated to a specific species after the binding assay, would provide for high throughput and a major advantage over existing systems.

### Summary of the Invention

In a first aspect of the invention, there is provided a method for simultaneous screening for interactions between chemical or biological species, comprising:
providing a first chemical or biological species, immobilized relative to a first surface, and a first oligonucleotide identifier independently associated with the first surface;
providing a second chemical or biological species, immobilized relative to a second surface;
allowing the first species to participate in a chemical or biological interaction with the second species;
determining participation of the first and second species in the interaction; and determining the identity of the first oligonucleotide identifier, thereby identifying the first species;
wherein the first surface is the surface of a nanoparticle.

In a second aspect of the invention, there is provided a kit comprising a plurality of gold nanoparticles, wherein the surface of each nanoparticle is completely covered by a self assembled monolayer that incorporates a carboxylate group, a metal chelate such as nitrilotriacetic acid, biotin, n-hydroxy-succinimide, glutathione, streptavidin, or fragment thereof and an oligonucleotide.

In a third aspect of the invention, there is provided a kit comprising a plurality of gold nanoparticles, wherein the surface of each nanoparticle is completely covered by a self assembled monolayer that incorporates thiols terminated with a binding partner of an affinity tag, and an oligonucleotide.

Preferred embodiments of the invention in any of its various aspects are as described below or as defined in the sub claims.

Other advantages, novel features, and objects of the invention will become apparent from the following detailed description of the invention when considered in conjunction with the accompanying drawings, which are schematic and which are not intended to be drawn to scale. In the figures, each identical or nearly identical component that is illustrated in various figures is represented by a single numeral. For purposes of clarity, not every component is labeled in every figure, nor is every component of each embodiment of the invention shown where illustration is not necessary to allow those of ordinary skill in the art to understand the invention.

### Brief Description of the Drawings

Fig. 1 illustrates schematically an embodiment of a colloid particle 140 adapted to bind essentially any chemical or biological species and also to bind an oligonucleotide identifier.
Fig. 2 illustrates schematically a chip including a plurality of spatially-addressable regions, each region having a chemical or biological species (putative binding species) and an oligonucleotide identifier.
Fig. 3 illustrates, schematically, another embodiment showing a chip to which one or more chemical or biological species are fastened.
Fig. 4 illustrates an oligonucleotide identifier of the invention adapted to be fastened to a surface, specifically via a self-assembled monolayer- forming species.
Fig. 5 illustrates identification of the polyamino acid tag of Figures 4-8, following separation from the surface of the colloid particle to which it had been fastened.
Fig. 6 illustrates a surface of a colloid particle to which is fastened an oligonucleotide identifier (Fig. 4) and a biological binding partner.
Fig. 7 illustrates biological binding between first and second biological binding partners attached to first and second colloid particles, respectively.
Fig. 8 illustrates separation of the oligonucleotide identifier of Fig. 6 from the surface of the colloid particle to which it had been fastened.
Fig. 9 illustrates an oligonucleotide identifier and a biological binding partner, each fastened to a surface of a colloid particle.
Fig. 10 illustrates two colloid particles, each carrying a biological species that biologically binds to the species of the other colloid particle, and each carrying a oligonucleotide identifier.
Fig. 11 illustrates binding of an interaction hybridization identifier to the combination of the oligonucleotide identifiers bound, respectively, to the colloid particles of Fig. 10.
Fig. 12 illustrates de-activating any non-hybridized oligonucleotide.
Fig. 13 illustrates the result of the step of Fig. 12.
Fig. 14 illustrates denaturization of the interaction hybridization identifier of Figures 11-13;
Fig. 15 illustrates identification of chimeric oligo solution and thereby identification of the oligonucleotide identifiers of Figures 10-13.
Fig. 16 shows ACV demonstration of enhanced electronic communication across a self-assembled monolayer, and redox signaling of protein immobilization to a cell surface, against a control.
Fig. 17 shows ACV analysis of protein/protein interaction as measured by binding of a colloid to a magnetic bead.
Fig. 18 illustrates how two binding partners can be detected through magnetic recruitment.
Fig. 19 illustrates a multiplexing apparatus for applying and releasing a magnetic force at multiple locations on a continuous surface.

### Detailed Description of the Invention

International patent application number PCT/US00/01997, filed 01/25/00 by Bamdad et al., entitled "Rapid and Sensitive Detection of Aberrant Protein Aggregation in Neurodegenerative Diseases" (published as WO 00/43791 on 07/27/00), International patent application number PCT/US00/01504, filed 01/21/00 by Bamdad, et al, entitled "Interaction of Colloid-Immobilized Species with Species on Non-Collodial Structures" (published as WO 00/43783 on 07/27/00), commonly-owned, copending U.S. patent application serial no. 09/602,778, filed 06/23/00 by Bamdad et al., entitled "Interaction of Colloid-Immobilized Species with Species on Non-Colloidal Structures"; and commonly-owned, copending U.S. patent application serial no. 09/631,818, filed 08/03/00 by Bamdad et al., entitled "Rapid and Sensitive Detection of Protein Aggregation".

"Small molecule", as used herein, means a molecule less than 5 kiloDalton, more typically less than 1 kiloDalton. As used herein, "small molecule" excludes proteins.

The term "candidate drug" as used herein, refers to any medicinal substance used in humans, animals, or plants. Encompassed within this definition are compound analogs, naturally occurring, synthetic and recombinant pharmaceuticals, hormones, antimicrobials, neurotransmitters, etc. This includes any substance or precursor (whether naturally occurring, synthetic or recombinant) which is to be evaluated for use as a drug for treatment of neurodegenerative disease, or other disease characterized by aberrant aggregation, or prevention thereof. Evaluation typically takes place through activity in an assay, such as the screening assays of the present invention.

A variety of types of particles can be used. For example, "fluid suspendable particle" means a particle that can be made to stay in suspension in a fluid in which it is used (typically an aqueous solution) by
itself, or can be maintained in solution by application of a magnetic field, an electromagnetic field, agitation such as stirring, shaking, vibrating, sonicating, centrifuging, vortexing, or the like. Examples include colloid particles, nanocrystals, and the like. A "nanoparticle" is a particle that can be fluid-suspendable, having a maximum cross-sectional dimension of no more than 500 nanometers, preferably no more than 250 nanometers. A "magnetically suspendable" particle is one that can be maintained in suspension in a fluid via application of a magnetic field. An electromagnetically-suspendable particle is one that can be maintained in suspension in a fluid by application of an electromagnetic field (e.g., a particle carrying a charge, or a particle modified to carry a charge). A "self-suspendable particle" is a particle that is of low enough size and/or mass that it will remain in suspension in a fluid in which it is used (typically an aqueous solution), without assistance of for example a magnetic field, for at least 1 hour. Other self-suspendable particles will remain in suspension, without assistance, for 5 hours, 1 day, 1 week, or even 1 month, in accordance with the invention.

"Proteins" and "peptides" are well-known terms in the art, and are not precisely defined in the art in terms of the number of amino acids that each includes. As used herein, these terms are given their ordinary meaning in the art. Generally, peptides are amino acid sequences of less than about 100 amino acids in length, but can include sequences of up to 300 amino acids. Proteins generally are considered to be molecules of at least 100 amino acids.

As used herein, a "metal binding tag" refers to a group of molecules that can become fastened to a metal that is coordinated by a chelate. Suitable groups of such molecules include amino acid sequences, typically from about 2 to about 10 amino acid residues. These include, but are not limited to, histidines and cysteines ("polyamino acid tags"). Such binding tags, when they include histidine, can be referred to as a "poly-histidine tract" or "histidine tag" or "HIS-tag", and can be present at either the amino- or carboxy-terminus, or at any exposed region, of a peptide or protein or nucleic acid. A poly-histidine tract of six to ten residues is preferred for use in the invention. The poly-histidine tract is also defined functionally as being a number of consecutive histidine residues added to a protein of interest which allows the affinity purification of the resulting protein on a metal chelate column, or the identification of a protein terminus through the interaction with another molecule (e.g. an antibody reactive with the HIS-tag).

"Affinity tag" is given its ordinary meaning in the art. Affinity tags include, for example, metal binding tags, GST (in GST/glutathione binding clip), and streptavidin (in biotin/streptavidin binding). At various locations herein specific affinity tags are described in connection with binding interactions.

As used herein, "chelate coordinating a metal" or metal coordinated by a chelate, refers to a metal coordinated by a chelating agent that does not fill all available coordination sites on the metal, leaving some coordination sites available for binding via a metal binding tag. U.S. Patent No. 5,620,850 of Bamdad, et al.
, describes exemplary chelates. Examples include nitrilotriacetic acid, 2,2'-bis(salicylideneamino)-6,6'-demethyldiphenyl, and 1,8-bis(a-pyridyl)-3,6-dithiaoctane, or the like.

"Signaling entity" means an entity that is capable of indicating its existence in a particular sample or at a particular location. Signaling entities can be those that are identifiable by the unaided human eye, those that may be invisible in isolation but may be detectable by the unaided human eye if in sufficient quantity (e.g., colloid particles), entities that absorb or emit electromagnetic radiation at a level or within a wavelength range such that they can be readily detected visibly (unaided or with a microscope including an electron microscope or the like), or spectroscopically, electroactive entities that can be detected electronically or electrochemically, such as redox-active molecules exhibiting a characteristic oxidation/reduction pattern upon exposure to appropriate activation energy ("electronic signaling entities"), or the like. Examples include optically active entities such as dyes, pigments, transition metal complexes, redox-active metal complexes, fluorescent or phosphorescent moieties (including, by definition, fluorescent or phosphorescent proteins such as green fluorescent protein (GFP), phosphorescent moieties), up-regulating phosphors, chemiluminescent entities, electrochemiluminescent entities, or enzyme-linked signaling moieties including horse radish peroxidase and alkaline phosphatase. "Precursors of signaling entities" are entities that by themselves may not have signaling capability but, upon chemical, electrochemical, electrical, magnetic, or physical interaction with another species, become signaling entities. An example includes a chromophore having the ability to emit radiation within a particular, detectable wavelength only upon chemical interaction with another molecule. Precursors of signaling entities are distinguishable from, but are included within the definition of, "signaling entities" as used herein. Another example of a signaling entity is a particle that is made up of material that possesses an inherent signaling capability, including those materials whose signaling capabilities requires excitation with external energy sources. A preferred electroactive molecule as a signaling entity of the invention is a metallocene. Metallocenes that can operate as electroactive signaling elements in accordance with the invention are known. One example of a particularly preferred electroactive molecule is one containing a ferrocene or a ferrocene derivative group or derivative, such as ferrocenyl thiol (C₃₅H₂₄FeS); however, other organic complexes of transitions metals are also contemplated as signaling elements.

As used herein, "fastened to or adapted to be fastened", in the context of a species relative to another species or to a surface of an article, means that the species is chemically or biochemically linked via covalent attachment, attachment via specific biological binding (e.g., biotin/streptavidin), coordinative bonding such as chelate/metal binding, or the like. For example, "fastened" in this context includes multiple chemical linkages, multiple chemical/biological linkages, etc., including, but not limited to, a binding species such as a peptide synthesized on a polystyrene bead, a binding species specifically biologically coupled to an antibody which is bound to a protein such as protein A, which is covalently attached to a bead, a binding species that forms a part (via genetic engineering) of a molecule such as GST or Phage, which in turn is specifically biologically bound to a binding partner covalently fastened to a surface (e.g., glutathione in the case of GST), etc. As another example, a moiety covalently linked to a thiol is adapted to be fastened to a gold surface since thiols bind gold covalently. Similarly, a species carrying a metal binding tag is adapted to be fastened to a surface that carries a molecule covalently attached to the surface (such as thiol/gold binding) which molecule also presents a chelate coordinating a metal. A species also is adapted to be fastened to a surface if a surface carries a particular nucleotide sequence, and the species includes a complementary nucleotide sequence.

"Covalently fastened" means fastened via nothing other than one or more covalent bonds. E.g. a species that is covalently coupled, via EDC/NHS chemistry, to a carboxylate-presenting alkyl thiol which is in turn fastened to a gold surface, is covalently fastened to that surface.

"Specifically fastened" or "adapted to be specifically fastened" means a species is chemically or biochemically linked to another specimen or to a surface as described above with respect to the definition of "fastened to or adapted to be fastened", but excluding all non-specific binding.

"Non-specific binding", as used herein, is given its ordinary meaning in the field of biochemistry.

"Colloids", as used herein, means nanoparticles, i.e. very small, self-suspendable or fluid-suspendable particles including those made of material that is, e.g., inorganic or organic, polymeric, ceramic, semiconductor, metallic (e.g. gold), non-metallic, crystalline, amorphous, or a combination. Typically, colloid particles used herein are of less than 250 nm cross section in any dimension, more typically less than 100 nm cross section in any dimension, and in most cases are of about 2-30 nm cross section. One class of colloids suitable for use is 10-30 nm in cross section, and another about 2-10 nm in cross section. As used herein this term includes the definition commonly used in the field of biochemistry.

A "moiety that can coordinate a metal", a used herein, means any molecule that can occupy at least two coordination sites on a metal atom, such as a metal binding tag or a chelate.

As used herein, a component that is "immobilized relative to" another component either is fastened to the other component or is indirectly fastened to the other component, e.g., by being fastened to a third component to which the other component also is fastened, or otherwise is translationally associated with the other component. For example, a signaling entity is immobilized relative to a binding species if the signaling entity is fastened to the binding species, is fastened to a colloid particle to which the binding species is fastened, is fastened to a dendrimer or polymer to which the binding species is fastened, etc. A colloid particle is immobilized relative to another colloid particle if a species fastened to the surface of the first colloid particle attaches to an entity, and a species on the surface of the second colloid particle attaches to the same entity, where the entity can be a single entity, a complex entity of multiple species, a cell, another particle, etc. Where a
species is described as immobilized relative to another entity (another species, a surface, etc.), it to be understood that the species can be fastened to the entity in some embodiments, where those of ordinary skill in the art would understand that it is possible for the species to be fastened to the entity.

"Diverse biological animals" means different animals, such as mouse and hamster, mouse and goat, etc.

The term "sample" refers to any cell, tissue, or fluid from a biological source (a "biological sample", or any other medium, biological or non-biological, that can advantageously be evaluated including, but not limited
to, a biological sample drawn from a human patient, a sample drawn from an animal, a sample drawn from food designed for human consumption, a sample including food designed for animal consumption such as livestock feed, milk, an organ donation sample, a sample of blood destined for a blood supply, a sample from a water supply, or the like. One example of a sample is a sample drawn from a human or animal to whom a candidate drug has been given to determine the efficacy of the drug.

A "sample suspected of containing" a particular component means a sample with respect to which the content of the component is unknown. For example, a fluid sample from a human suspected of having a disease, such as a neurodegenerative disease or a non-neurodegenerative disease, but not known to have the disease, defines a sample suspected of containing neurodegenerative disease aggregate-forming species. "Sample" in this context includes naturally-occurring samples, such as physiological samples from humans or other animals, samples from food, livestock feed, etc., as well as "structurally predetermined samples", which are defined herein to mean samples, the chemical or biological sequence or structure of which is a predetermined structure used in an assay designed to test whether the structure is associated with a particular process such as a neurodegenerative disease. For example, a "structurally predetermined sample" includes a peptide sequence, random peptide sequence in a phage display library, and the like. Typical samples taken from humans or other animals include cells, blood, urine, ocular fluid, saliva, cerebro-spinal fluid, fluid or other samples from tonsils, lymph nodes, needle biopsies, etc.

As used herein, "metal binding tag/metal/chelate linkage" defines a linkage between first and second species in which a first species is immobilized relative to a metal binding tag and a second species is immobilized relative to a chelate, where the chelate coordinates a metal to which the metal binding tag is also coordinated. U.S. Patent No. 5,620,850 of Bamdad, et al. describes exemplary linkages.

The term "biological binding" refers to the interaction between a corresponding pair of molecules that exhibit mutual affinity or binding capacity, typically specific or non-specific binding or interaction, including biochemical, physiological, and/or pharmaceutical interactions. Biological binding defines a type of interaction that occurs between pairs of molecules including proteins, nucleic acids, glycoproteins, carbohydrates, hormones and the like. Specific examples include antibody/antigen, antibody/hapten, enzyme/substrate, enzyme/inhibitor, enzyme/cofactor, binding protein/substrate, carrier protein/substrate, lectin/carbohydrate, receptor/hormone, receptor/effector, complementary strands of nucleic acid, protein/nucleic acid repressor/inducer, ligand/cell surface receptor, virus/ligand, etc.

The term "binding partner" refers to a molecule that can undergo binding with a particular molecule. Members of pairs of molecules that can undergo biological binding, as exemplified above, are examples. For example, Protein A is a binding partner of the biological molecule IgG, and vice versa.

The term "determining" refers to quantitative or qualitative analysis of a species via, for example, spectroscopy, ellipsometry, piezoelectric measurement, immunoassay, electrochemical measurement, and the like. "Determining" also means detecting or quantifying interaction between species, e.g. detection of binding between two species.

The term "self-assembled monolayer" (SAM) refers to a relatively ordered assembly of molecules spontaneously chemisorbed on a surface, in which the molecules are oriented approximately parallel to each other and roughly perpendicular to the surface. Each of the molecules includes a functional group that adheres to the surface, and a portion that interacts with neighboring molecules in the monolayer to form the relatively ordered array. A wide variety of SAMs can be used:
, on a wide variety of surfaces, to present desired species such as binding partners, signaling entities, and the like at a surface of an article such as an electrode, colloid particle, or the like. Those of ordinary skill in the art can select from among a wide variety of surfaces, functional groups, spacer moieties, etc. for forming SAMs. An exemplary description can be found in U.S. Patent No. 5,620,850. See also Laibinis, P. E.; Hickman, J.; Wrighton, M. S.; Whitesides, G. M. Science 245, 845 (1989), Bain, C.; Evall, J.; Whitesides, G. M. J. Am. Chem. Soc. 111, 7155-7164 (1989), Bain, C.; Whitesides, G. M. J. Am. Chem. Soc. 111, 7164-7175 (1989).

The formation of SAMs on fluid-suspendable particles such as colloid particles is described in U.S. Patent Application Serial No. 09/602,778, filed June 23, 2000, entitled "Interaction of Colloid Immobilized Species with on Non-Colloidal Structures", by Bamdad, et al.

Also described is the use of self-assembled monolayers (SAMs) on surfaces, such as surfaces of colloid particles, and articles such as colloid particles having surfaces coated with SAMs.

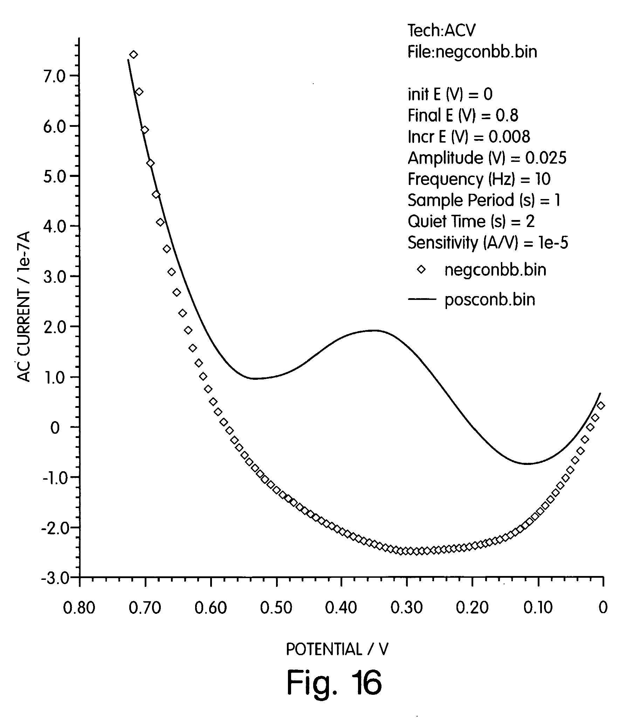

Preferably, SAMs formed completely of synthetic molecules completely cover a surface or a region of a surface, e.g. completely cover the surface of a colloid particle. "Synthetic molecule", in this context, means a molecule that is not naturally occurring, rather, one synthesized under the direction of human or human-created or human-directed control. "Completely cover" in this context, means that there is no portion of the surface or region that directly contacts a protein, antibody, or other species that prevents complete, direct coverage with the SAM. I.e., preferably the surface or region includes, across its entirety, a SAM consisting completely of non-naturally-occurring molecules (i.e. synthetic molecules). The SAM can be made up completely of SAM-forming species that form close-packed SAMs at surfaces, or these species in combination with molecular wires or other species able to promote electronic communication through the SAM (including defect-promoting species able to participate in a SAM), or other species able to participate in a SAM, and any combination of these. Preferably, all of the species that participate in the SAM include a functionality that binds, optionally covalently, to the surface, such as a thiol which will bind to a gold surface covalently. A self-assembled monolayer on a surface
, can be comprised of a mixture of species (e.g. thiol species when gold is the surface) that can present (expose) essentially any chemical or biological functionality. For example, they can include tri-ethylene glycol-terminated species (e.g. tri-ethylene glycol-terminated thiols) to resist non-specific adsorption, and other species (e.g. thiols) terminating in a binding partner of an affinity tag, e.g. terminating in a chelate that can coordinate a metal such as nitrilotriacetic acid which, when in complex with nickel atoms, captures a metal binding tagged-species such as a histidine-tagged binding species. Also provided is a method for rigorously controlling the concentration of essentially any chemical or biological species presented on a colloid surface or any other surface. Without this rigorous control over peptide density on each colloid particle, co-immobilized peptides would readily aggregate with each other to form micro-hydrophobic-domains that would catalyze colloid-colloid aggregation in the absence of aggregate-forming species present in a sample. This is an advantage over existing colloid agglutination assays. Preferably, the self-assembled
monolayer is formed on gold colloid particles. Self-assembled monolayers can be made to be electrically conductive. As a working example, fig. 16 shows ACV demonstration of enhanced electronic communication across a self-assembled monolayer, and redox signaling of protein immobilization to a cell surface, against a control.

A "self-assembled monolayer-forming species" comprises a species that, when exposed to an appropriate surface with other, like species, e.g. provided with like species in an appropriate solution and exposed to an appropriate surface, will spontaneously form a self-assembled monolayer on the surface.

A species "able to integrate into a self-assembled monolayer" (which can be a self-assembled monolayer forming species) is a species having a chemical functionality favoring participation in a self-assembled monolayer comprising the species and other, self-assembled monolayer-forming species with which it is not chemically incompatible. For example, the species may include a functional group selected to adhere to a surface on which the self-assembled monolayer is formed, and may include a remainder portion that may be approximately linear (not highly-branched), but which does not facilitate close packing. Molecules including a significant amount of unsaturation, for example a series of interconnected aromatic rings, are examples. Such a species may or may not be a self-assembled monolayer-forming species. Typically, species that are able to integrate into a self-assembled monolayer but are not able themselves to form a self-assembled monolayer will be able to participate in formation of and integrate into a self-assembled monolayer when present in an amount of up to about 50% as a percentage of overall species including chemically-compatible self-assembled monolayer-forming species.

The term "self-assembled mixed monolayer" refers to a heterogeneous self-assembled monolayer, that is, one made up of a relatively ordered assembly of at least two different molecules.

"Molecular wires" as used herein, means wires that enhance the ability for a fluid encountering a SAM-coated electrode to communicate electrically with the electrode. This includes electrically conductive molecules or molecules that can cause defects in the SAM allowing communication with the electrode. Anon-limiting list of additional molecular wires includes 2-mercaptopyridine, 2-mercaptobenzothiazole, dithiothreitol, 1, 2-benzenedithiol, 1, 2-benzenedimethanethiol, benzene-ethanethiol, and 2-mercaptoethylether. Conductivity of a monolayer can also be enhanced by the addition of molecules that promote conductivity in the plane of the electrode. Conducting SAMs can be composed of, but are not limited to: 1) poly (ethynylphenyl) chains terminated with a sulfur; 2) an alkyl thiol terminated with a benzene ring; 3) an alkyl thiol terminated with a DNA base; 4) any sulfur terminated species that packs poorly into a monolayer; 5) all of the above plus or minus alkyl thiol spacer molecules terminated with either ethylene glycol units or methyl groups to inhibit non specific adsorption. A variety of molecules can be used for this purpose, including but not limited to poly (ethynylphenyl thiol) (i.e. C₁₆H₁₀S), referred to herein as MF1.: 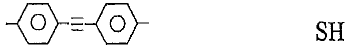 Thiols are described because of their affinity for gold in ready formation of a SAM.

Other molecules can be substituted for thiols as known in the art from U.S. Patent No. 5,620,820, and other references. Molecular wires typically conduct electronically or, because of their bulk or other conformation, creates defects in an otherwise relatively tightly-packed SAM to prevent the SAM from tightly sealing the surface against fluids to which it is exposed. The molecular wire causes disruption of the tightly-packed self-assembled structure, thereby defining defects that allow fluid to which the surface is exposed to communicate electrically with the surface. In this context, the fluid communicates electrically with the surface by contacting the surface or coming in close enough proximity to the surface that electronic communication via tunneling or the like, can occur.

A "chimeric oligo solution" is an oligonucleotide sequence, such as DNA, that is simultaneously complimentary to two oligonucleotide identifiers each of which corresponds to two different binding partners. A complete set of chimeric oligo solutions represents a set of all possible combinations of interacting binding partners in any given procedure.

Also provided are methods by which a large number of proteins, such as those encoded by entire genomes of an organism, can be simultaneously tested for interaction with any known or unknown component of a genome or with any chemical species. Also provided are methods for detecting interactions involving proteins in which the nucleic acids, which encode them, are immediately available once the protein has been selected as an interacting species.

Also provided are methods for detecting interactions between genetically encoded species and chemical species in order to identify new affinity reagents for biological and biochemical studies. Much of the following description involves a variety of methods, compositions and species, and articles for monitoring (detecting) interactions between chemical or biological species including techniques useful for drug screening. Major features
include the following. Tools for proteomic studies including protein chips and particles for signaling interactions, and multi-particle systems such as two-particle systems. In multi-particle systems one particle can be a recruitable particle and the other particle can carry a binding partner of an agent presented by the recruitable particle and can also be a signaling entity or carry an auxiliary signaling entity. Another major area involves cell studies, especially techniques involving interactions between ligands and cell surface proteins and receptors. Discovery and therapeutics involving drugs that can effect these interactions also is described, with an emphasis on drug therapy involving angiogenesis. Specifically, cell receptor/ligand interactions that can inhibit or promote angiogenesis are described. Another area involves detecting proteins, either in solution or on the surfaces of intact cells, for diagnostic purposes.

A major disadvantage of existing **in vitro* binding assays is that they are not compatible with high throughput. Proteins must be sequentially tested in pair-wise binding assays because: 1) proteins cannot be amplified as nucleic acids can, making identification by sequencing after a binding assay difficult or impossible; therefore, the identity of each putative binding partner must be carefully tracked; 2) typical binding assays produce a single type of signal so that each pair to be tested must be kept in isolation so that a positive signal can be assigned to the appropriate binding partners.

These problems are solved by providing
a convenient method of tracking proteins that have been pooled, through the use of coding tags (identifiers) that are linked to putative binding species, which include proteins as well as chemical species. Methods detail techniques for
"connecting" a coding identifier to a biological or chemical species via co-immobilization on a common surface, which preferably is the surface
of a particle. Preferably, an oligo is used to identify the
biological or chemical species, wherein the 4-bit code of a DNA uniquely identifies the co-immobilized species. Preferably, the biological species
under study is a protein that is expressed off of the encoding DNA, which uniquely identifies it. Also described are methods that facilitate the attachment of the identifier to a surface to which the biological or chemical species is also attached. Preferably, an expressed protein and its encoding plasmid DNA are
attached to a common particle via an affinity tag on the protein binding to a metal chelate on the particle and a DNA recognition motif contained within the plasmid binding to DNA-binding proteins on the particle surface.

Further described are high throughput methods for detecting and selecting interacting partners, then rapidly identifying the interacting partners.

A variety of techniques and components associated with various assays, kits, detection methods, etc. are described below. It is to be understood that the techniques
involving oligonucleotide identifiers and proteins can be used in conjunction with any of the specific assays described herein, and these assays are provided by way of example only, as oligonucleotide identifier techniques
can be used in conjunction with essentially any biological or chemical binding assay. Oligonucleotide identifier techniques are particularly well-suited to assays involving particles, beads, chips and colloids, in order to rapidly identify interacting protein partners from a pool of putative interactors, which are described below.

Those of ordinary skill in the art will clearly understand where, in the following description, oligonucleotide identifier techniques can be used, and where oligonucleotide identifier techniques can be used in essentially any assay technique.

Contemplated is interaction between chemical
or biological agents for analysis, drug screening, or the like.

Also included is analyzing and/or inhibiting protein-protein interactions, protein-chemical species interactions, ligand-nucleic acid interactions, ligand-receptor interactions, including but not limited to ligands on intact cells (growing on an electrode, or in solution or in suspension). Also contemplated is
the use of drug candidates, known or putative ligands, and small molecule drug libraries.

Also involved are oligonucleotide identifiers, which by definition include any number of bases (nucleotides), in which the 4-bit nucleic acid code is used to form a sequence that uniquely identifies some natural or synthetic material. This includes natural or synthetic nucleotide sequences, or derivatives of nucleic acids (including DNA and thiol-modified DNA, nucleotides fastened to polymer backbones, etc) that are adapted to be fastened to surfaces, that also can carry potential chemical or biological binding partners. The oligonucleotide identifiers can be short DNA sequences, for example from about 2 to about 20 bases in length, preferably from about 6 to about 12 bases in length. Longer oligonucleotide identifiers can be used as well, for example those of up to 50, or 100, or several hundred bases in length.

An oligonucleotide identifier is attached to a surface to which a corresponding chemical or biological agent also is attached and which it will uniquely identify. The surface can be essentially any surface useful in chemical or biological analysis, including all surfaces described above, such as surfaces of particles such as fluid-suspendable particles or non-suspendable particles, larger surfaces such as those of chips, microarray chips, surfaces involved in electronic detection assays, cell surfaces etc. For example, the surface may be the surface of a colloid, where each colloid or set of colloids displays a single binding species and a single oligonucleotide identifier. Alternatively, the surface may be the surface of a spatially addressable array chip where multiple pairs of agents and identifiers are fastened in relatively close proximity to each other, but separated to the extent that they can be individually formed and analyzed. Each distinct spatial address of the chip displays a potential binding partner and an identifying oligonucleotide identifier nearby.

Species of interest, which are surface-immobilized relative to their unique encoding identifiers, are allowed to interact with other species in solution or attached to other surfaces or particles. Interacting species are isolated using any of a variety of techniques. The identities of the interacting species are then rapidly determined by sequencing, hybridizing, or otherwise determining the sequence of the attached identifier.

Surfaces to which oligonucleotide identifiers are fastened or adapted to be fastened also can carry immobilized signaling entities such as those described herein and in International Pat. Apl. No: PCT/US01/40801, filed 05/25/01, entitled, "Electroactive Surface-Confinable Molecules", by Bamdad, et al.
, as well as other documents
. As will be apparent from the description below, particles such as colloid particles can participate in oligonucleotide-identified interactions and may or may not carry auxiliary signaling entities. For example, colloid particles are used that are free of auxiliary signaling entities where the colloid particles themselves serve as signaling entities via color change upon agglomeration.

Colloid particles or other surfaces carry auxiliary signaling entities such as fluorescent markers (optionally different fluorescent markers at different wavelengths for different particles), electroactive species such as ferrocenes (optionally different ferrocenes with different oxidation/reduction potentials on different articles), etc.

Fastening of oligonucleotide identifiers and chemical or biological species that may be binding partners to surfaces can be carried out according to any technique known in the art. Preferred techniques involve the use of self-assembled monolayers on surfaces. Self-assembled monolayer-forming species, or species able to integrate into a self-assembled monolayers can include chemical or biological species to be studied or oligonucleotide identifiers, and can thereby be incorporated into SAMs. Alternatively, species able to form or integrate into SAMs can include linkers for attachment to chemical or biological species or oligonucleotide identifiers, and the oligonucleotide can be attached to the surface after SAM formation via the linkers. Such linkers can include affinity tags or species that bind to affinity tags, species suitable for EDC/NHS coupling, oligonucleotide linkers, biotin-streptavidin interaction, species that can participate in DNA ligation techniques etc. Examples of DNA ligation techniques include incorporation of a specific oligonucleotide sequence into a SAM, that encodes for a restriction site, and ligating a second oligonucleotide sequence, terminated in the same restriction site, to the original oligonucleotide sequence. An example of a commonly used ligase is T4 Ligase. Alternatively, a blunt-end oligonucleotide could be added using blunt-end ligation techniques. Preferred species for use with affinity tags include metals coordinated by chelates, for use with polyamino acid tags. For example, surfaces can be coated with SAMs exposing metals coordinated by chelates, and chemical or biological species, or oligonucleotide identifiers, can carry polyamino acid tags for coordination to the metal thereby linking the chemical or biological species, or oligonucleotide identifier, to the surface. Different chemistries, or the same chemistry can be used for linkage of any species or identifiers involved in the invention to a single surface.

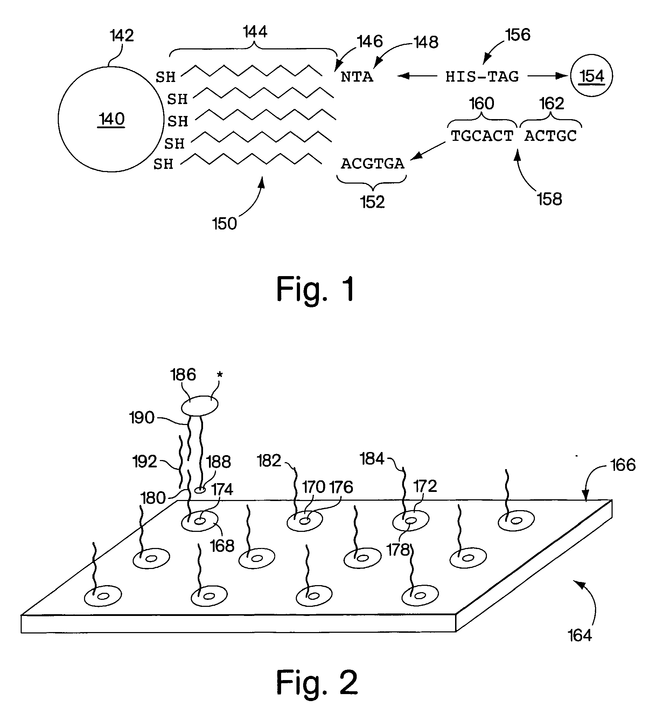

Referring now to Fig. 1, a surface of a colloid particle 140 adapted to bind essentially any chemical or biological species and also to bind an oligonucleotide identifier is illustrated schematically. Colloid 140 includes a surface 142 upon which is a SAM 144. SAM 144 is only partially illustrated - the SAM preferably will completely coat the surface 142. SAM 144 includes one species 146 that exposes, away from surface 142, terminating in a chelate able to coordinate a metal, or a chelate coordinating a metal, 148 (represented in the figure as NTA, nitrilotriacetic acid). A chemical or biological species 154, comprising a polyamino acid tag 156, when exposed to colloid 140, will fasten to chelate/metal 148.

SAM 144 also includes a species 150 comprising an oligonucleotide linker 152. An oligonucleotide species 158, including a linker portion 160 that is the complement of linker 152, and a section 162 defining an oligonucleotide identifier, when exposed to colloid particle 140 will fasten thereto. Thus, a plurality of colloid particles 140, each including an identically-derivatized surface can be provided as a kit. In an assay, different chemical or biological species 154 can be attached to one set of colloid particles to which is attached a unique oligonucleotide identifier 162. Different batches of colloid particles can carry different chemical or biological species and corresponding oligonucleotide identifiers. Of course, colloid particles can include solely species 146 where chemical or biological species and oligonucleotide identifiers each include polyamino acid tags 156, or colloid particles can include solely species 150 where both the chemical or biological species, and the oligonucleotide identifier, each includes an oligonucleotide linker 160. That is, oligonucleotide identifiers that uniquely identify a species attached to a surface need not be directly attached to the common surface. For example, for convenience, surfaces may be derivatized with a universal oligonucleotide. A second oligonucleotide comprised of a portion that is complementary to the universal DNA sequence, which is directly attached to the surface, and a second portion which uniquely identifies a chemical or biological species that is also attached to the surface.

The arrangement of Fig. 1 can be used to provide a set of colloid particles is provided, each carrying an immobilized chemical or biological species such as a biological binding partner (e.g., a protein or small molecule), and each an immobilized oligonucleotide identifier. A record is made of the sequence of the identifier, and the identity of the chemical or biological species (potential binding partner) immobilized to the same colloid particles as the identifier. As an example of procedure, sets of colloids each bearing a distinct species are pooled together in solution and allowed to interact. This next step is an alternative to separating out interacting particle-immobilized species, then sequentially releasing and sequencing the attached oligo identifiers in order to identify interacting species. A set of oligos is added that we call "chimeric oligo solutions". Each DNA strand in this set is comprised of a chimeric sequence that is complementary to two oligonucleotide identifiers. The entire set of "chimeric oligo solutions" would contain chimeric sequences that represent all possible solutions to the problem of which species interact with each other. These "chimeric oligo solutions" are then incubated with the colloids that present putative binding partners and attached oligo identifiers. The "chimeric oligo solutions" that have simultaneously hybridized to oligonucleotide identifiers on two sets of colloids that bear interacting partners, identify which putative binding species interact with each other. The hybridized "chimeric oligo solutions" need to be separated from oligos that remain free in solution as well as from oligos that have hybridized to only one sequence identifier. Free oligos are easily removed by pelleting the colloids and discarding the supernatant. Chimeric oligo solutions that are hybridized to only one sequence identifier can be distinguished from those hybridized to two enzymatically digesting the free end with enzymes that degrade single stranded nucleic acid strands. Chimeric oligosolutions are then released from the particles by any one of a number of methods including dissociation by heated water, chemical release, etc. The chimeric oligosolutions are then sequenced to reveal the identity of the interacting partners. The chimeric oligosolutions can also be enzymatically amplified (such as by PCR) prior to the sequencing step.

Preferably, an oligonucleotide identifier is involved that uniquely identifies a protein wherein the oligonucleotide identifier is the very sequence that encodes the protein. Both the expressed protein and the oligonucleotide identifier (e.g. DNA) that encoded it are immobilized relative to each other, e.g. each is immobilized relative to a linker species, so that the protein is presented for binding studies and its oligonucleotide identifier is retrievable after selection of interacting pairs of proteins.

The oligonucleotide identifier can be in essentially any form, e.g. plasmid form, as in a protein expression vector, or in a linear form, such as nucleic acids strands that are generated by a polymerase chain reaction (PCR). PCR generated fragments can be engineered to include sequences, such as a start site of transcription, to promote protein expression then translation. These nucleic acid templates are especially well suited for *in vitro,* cell-free protein expression systems such as the Rapid Translation System (RTS) sold by Roche Diagnostics.

A protein may be
expressed from an expression vector or template. The solution in which the protein is expressed will then contain both the expressed protein of interest (which can be a putative binding partner) and the DNA that encoded the protein (which will serve as the oligonucleotide identifier). Both the protein of interest and the oligonucleotide identifier that encodes the protein can be immobilized to a common linker species by a variety of methods. Where the linker species is an article, the oligonucleotide identifier can be immobilized relative to the surface of the article by a variety of methods as described herein. E.g., the surface can be derivatized to present both a moiety to facilitate the attachment of the protein of interest as well as a moiety to facilitate the attachment of the oligonucleotide identifier, as described herein. The protein can be expressed with an affinity tag from a DNA template that contains a convenient functionality that facilitates the DNA's attachment to a surface that also presents binding partners of the protein's affinity tag. For example, an affinity tagged protein can be expressed off a DNA template that bears biotin. When exposed to a surface bearing both a metal binding tag/metal/chelate linkage and streptavidin, both gene (a section of the oligonucleotide identifier) and gene product (protein) will be captured and presented on the common surface. A convenient approach is to express a histidine-tagged protein from a biotinylated template. Surfaces, including particles, such as colloids, are coated with heterologous SAMs bearing both NTA-nickel (the binding partner of the histidine tag) and biotin. The surfaces are first exposed to streptavidin, which has four binding sites for biotin. This surface will then capture and present the histidine tagged gene product as well as its encoding DNA sequence. As another example, proteins of interest fused to glutathione-S-transferase (GST) can be attached to entities or articles presenting glutathione. Alternatively, the protein of interest can be expressed as a fusion protein with thioredoxin, then attached to a surface that presents a binding partner of thioredoxin. GST and thioredoxin serve a dual purpose. First, these proteins provide a convenient affinity tag for the protein of interest. Secondly, proteins commonly used as fusion partners increase the solubility of the expressed protein of interest and thus increase the effective concentration of the protein.

In another technique for forming an oligonucleotide identifier immobilized relative to a protein for which is encodes, via a linker species, the oligonucleotide identifier may also contain a binding site for a DNA/RNA-binding protein. This sequence is preferably inserted downstream of the gene to be expressed. The protein that binds to the binding site on the oligonucleotide identifier can then be immobilized at a surface (e.g. on a particle) which also presents a moiety for the attachment of the expressed protein (e.g. NTA/nickel). Preferably, a nucleic acid binding protein is immobilized on the surface and used to capture the oligonucleotide identifier. Nucleic acid binding proteins bind to DNA or RNA by recognizing either a specific nucleic acid sequence motif or by recognizing a tertiary structure. For example, a common DNA-binding yeast protein is Gal4. It binds as a dimer to a specific sequence of double stranded DNA, while single stranded binding protein binds to single stranded DNA. Other proteins bind to structural elements of DNA or RNA, such as binding to cruciform DNA, hairpins or to specific RNA loops.

Ideally, the protein binding site sequences inserted into the template that encodes the protein of interest are recognition motifs for a protein from an organism distinct from that of the expression system. For example, if the protein is expressed using an E. *coli* based system, then the oligonucleotide identifier is attached to the common surface by binding to a yeast protein, such as Gal4. In this way, the protein expression system does not include or produce extraneous proteins that would compete for the cognate binding site on the oligonucleotide identifier.

In an alternative approach, the protein of interest is expressed (by an oligonucleotide identifier) as a fusion protein with a protein fragment that has a binding partner that can be attached to the oligonucleotide identifier. This provides a method for attaching the oligonucleotide identifier to the protein of interest. There are a variety of proteins or fragments of proteins that can be genetically fused to the protein of interest, which also bind to small molecules which can be used to modify DNA. Short strands of DNA that are so modified can be used as primers in PCR reactions to produce expression templates from which fusion proteins that capture their own oligonucleotide identifier can be produced.

For example, a PCR product that contains sequences that encode the protein of interest as well as sequences that encode streptavidin can be generated using biotinylated primers. The resultant PCR product, which will also include elements necessary for transcription/translation, serves as a template for protein expression and also yields an oligonucleotide identifier that has been adapted to be fastened to the protein expressed via the biotin-streptavidin linkage. If desired, the streptavidin may be expressed as a fragment that only binds one biotin. Additionally, the fusion protein which contains streptavidin and the protein of interest may also contain an affinity tag to facilitate attachment of the protein/oligonucleotide identifier to a common surface or particle.

In another example, the protein of interest is expressed as a fusion protein with a DNA-binding protein, such as LexA, from an expression vector or template that contains DNA-binding sites for LexA. In this way, the gene product and gene are linked to each other.

To facilitate the detection of binding events between proteins of interest, one of the proteins can be modified with a signaling entity. One way to accomplish this is by direct attachment of the signaling entity to one of the proteins of interest. Another way is to genetically fuse the protein of interest to a protein that has a signaling capability. For example, a protein of interest can be expressed as a green fluorescent protein (GFP) fusion protein.

Alternatively, the proteins of interest have signaling capability when they are co-immobilized with a signaling entity on a common surface. For example, a protein of interest can be attached to a polymer to which a signaling entity such as Ru complex has also been attached. Binding partners are attached to recruitable particles such as magnetic beads and then detected by ECL methods. Similarly, proteins of interest can be co-immobilized on particles that also present signaling entities. For example a protein of interest can be attached to a particle or colloid that also presents signaling entities such as fluorescent or phosphorescent or redox-active moieties to facilitate optical or electrochemical detection, respectively.

Preferably, the oligonucleotide identifier that encodes the protein of interest is generated such that it also possesses signaling capabilities. For example, the DNA expression template can be generated by PCR using primers in which at least some of the bases have been chemically modified with redox-active moieties such as ferrocene derivatives. The protein of interest and the oligonucleotide identifier (expression template) are then attached to a common surface, such as a colloid particle. The particles are mixed with putative binding partners attached to magnetic beads, and magnetically drawn to a sensing electrode where the presence of the redox-active molecule, hence the interaction, is detected. Alternatively, bases modified with a signaling functionality can be used in the polymerase chain reaction to generate an expression template that can signal. Similarly, the protein of interest is expressed from a DNA template that has been modified with a fluorescent or phosphorescent moiety.

In one example of synthesis and use, a nucleic acid is generated that encodes a histidine-tagged protein of interest (for attachment of the protein to a surface that displays an NTA-Ni⁺⁺ moiety) and that also contains a Gal4 binding site for attachment of the oligonucleotide identifier to the common surface. The protein is preferably expressed in a cell-free system to minimize levels of background DNA and proteins. After protein expression, the reaction mixture is incubated with particles (e.g. colloids or beads) that present metal/chelate (e.g. NTA-Ni) moieties and Gal4 for fastening of the protein and oligonucleotide identifier, respectively. The particle-immobilized NTA-Ni⁺⁺ moieties capture and present the protein of interest (via the binding tag) and the particle-immobilized Gal4 captures the DNA that encodes the expressed protein, because it also contained Gal4 recognition sites. In this way an identifying oligonucleotide identifier has been immobilized relative to the expressed protein.

These particles can then be used in a variety of assay formats. The following assay is compatible with detecting protein-protein interactions involving large numbers of possible binding partners. A portion of the putative binding partners is attached to a set of colloids, which also bear Gal4 for the attachment of the nucleic acid, which encoded the protein. The colloids also carry signaling entities. The signaling entities can be electroactive, electrochemiluminescent, optical, etc. A second set of putative binding partners is attached to magnetic particles. Large numbers of magnetic particles and colloidal particles are mixed together to allow binding to occur between proteins immobilized relative to the beads and particles. A magnetic field is then used to collect the magnetic particles at a sensing location where the signals carried on the colloids can be detected.

As described, a gene product (protein) and the gene (oligonucleotide identifier) are connected to each other via attachment to a linker species. Protein recognition motifs (DNA or RNA sequences) are inserted into an expression (or translation) vector up- or downstream of the sequences that encode the gene of interest. These DNA recognition motifs facilitate attachment of the gene to a surface or particle presenting the cognate DNA-binding protein. The surface also presents a moiety to facilitate attachment of the gene product. For example, surfaces that present both NTA-Ni and Gal4 will capture histidine-tagged proteins and DNA that bears Gal4 recognition sequences (motifs). The nucleic acids referenced here can be any nucleic acid encoding the gene of interest and having the ability to promote transcription or translation, including: a protein expression vector (plasmid); a linear piece of double stranded DNA, such as a PCR product, including those designed for *in vitro* protein expression or translation.

The nucleic acid that encodes the gene of interest is chemically modified to facilitate its attachment to a surface or a particle. For example, a protein is expressed in a cell-free expression system off of a biotinylated PCR product. If the gene of interest has been modified with an affinity tag such as GST, both the gene product and the gene can be attached to a surface that presents both glutathione and streptavidin (or other avidin derivative).

The gene of interest is expressed as a fusion protein with a protein that can be readily attached to the encoding gene. For example, the gene of interest is expressed as a LexA fusion protein and the DNA template or plasmid, used for protein expression or translation, also contains DNA binding sites for LexA. As another example, the gene of interest is expressed as a streptavidin fusion protein and the DNA template used for protein expression or translation, is biotinylated.

Also described is the investigation of interactions between chemical or biological species, such as protein-protein binding interactions, on a very large scale. Large numbers of potential binding interactions can be investigated in a single solution. Once many potential binding partners are brought together, they must be separated to identify which were involved in binding interactions. The following is a description of techniques for separating species that have been involved in interaction from species that have not.

As one example, magnetic *in situ* selection/dilution can be used, as described in aU.S. patent application, filed 10/03/01, entitled "Magnetic In Situ Dilution", by Bamdad.

The magnetic *in situ* selection/dilution technique can be used in connection with any format in which a potential binding partner is immobilized relative to a particle that can be drawn to a magnet, where once the particle is drawn to the magnet, whether the species has been involved in a binding interaction can be detected. For example, potential binding partners can be immobilized relative to magnetic particles, and species that might bind with these species can be labeled with signaling entities such that once a particle is drawn to a magnet the presence or absence of the signaling entity in proximity of the magnet can be detected. For example, as described in the above-noted patent application: an array of electrodes can be provided on a surface, each electrode individually addressable, and each electrode accompanied by a corresponding individually-addressable magnet. A plurality of magnetic beads can be provided, each carrying an immobilized potential biding partner. A plurality of species that are putative binding partners of the binding partners immobilized on the magnetic beads can be provided, each of these species immobilized relative to a signaling entity. The binding partners on the beads can be the same or different, and the species that are their putative binding partners can be the same or different. Mixing the magnetic bead-immobilized binding partners with the species that are their putative binding partners may result in some magnetic beads immobilized relative to signaling entities (where binding has occurred between putative partners) and other magnetic beads that are not immobilized relative to signaling entities. Following mixture, within a fluid medium, of the putative binding partners, and optionally without any wash step, the solution can be exposed to the electrode array. Beads can be magnetically drawn to electrodes and determination can be made as to which electrodes have not drawn beads immobilized to signaling entities (for example, optically where the signaling entities are optical signaling entities or electrochemically where the electrodes corresponding to the magnetic beads generate a signal determinative of whether a signaling entity is or is not in proximity). Following this, individual magnetic regions that have not attracted beads carrying immobilized signaling entities are de-magnetized, releasing the beads, which are washed away. Magnets at which beads carrying signaling entities have been attracted remain magnetized during this washing and removal step. Subsequent to removal, all magnets are released and the process is continued until, statistically, only one magnetic bead exists for each magnet. At this point, after a magnetic attraction step, each magnet that has not attracted a bead carrying an immobilized signaling entity is released, and all remaining magnets will have attracted only magnetic beads carrying a pair of binding partners indicated by the presence of a signaling entity at the magnet. At this point, these species are released and the identity of binding partners is identified via identification of oligonucleotide identifiers described herein. This is the technique described generally in the above-identified U.S. patent applications.

As a specific example, a first set of gene products (proteins) and their respective genes (oligonucleotide identifiers) are attached to magnetic beads. A second set of gene products and their respective genes are attached to particles, such as colloids, that also bear electronic signaling moieties. The two particle populations are incubated together in solution and binding interactions between proteins immobilized on different particles are allowed to occur. When an interaction takes place between a protein on a magnetic bead and a protein on a colloid bearing electroactive groups, the recruitable particle (magnetic bead) becomes connected to the signaling particle. A magnetic field draws the complex to a sensing electrode where an electronic signal is transduced. The sensing electrode is an array of electrodes configured such that the recruitment of magnetic beads to each electrode pad is individually controlled. This can be accomplished by interfacing each electrode pad with an individually controllable electromagnet. That is, an array of individually-addressable electromagnets is fabricated (using techniques known to those of ordinary skill in the art). Where the electronic signaling entity is a redox-active molecule such as ferrocene, an electrode is associated with each magnet such that when beads are drawn to a magnet, the electrode associated with the magnet is cycled and if a redox-active signaling entity is immobilized relative to the bead (via binding partner interaction), then the redox signature of the signaling entity will be detected.

Another example involves optical selection. In this technique, binding partners identified by immobilized oligonucleotide identifiers, that participate in binding interactions are optically detected by any technique described herein such as visual or automatic observation of color change upon colloid-colloid aggregation, colloid "decoration" of beads upon aggregation, etc. As a specific example, interacting partners, attached to different particle types, can be optically selected by a variety of methods. A first set of gene products (proteins that are putative binding partners) and genes (oligonucleotide identifiers) can be attached to non-magnetic beads. A second set of gene products can be directly or indirectly modified with fluorescent or phosphorescent moieties. For example, the second set of gene products can be attached to colloids that also bear fluorescent or phosphorescent moieties. The interaction between bead-immobilized and colloid-immobilized proteins causes the agglomeration of the fluorescent or phosphorescent colloids onto the larger bead and renders it (the bead) detectable and identifiable as a bead that presents a protein that is participating in an interaction. In an alternative approach, the second set of gene products is expressed as a fusion protein with a DNA binding protein or domain and the is expressed or translated off of a fluorescent or phosphorescently labeled nucleic acid. As in the previous case, the interaction between bead-immobilized proteins and the fusion proteins causes the agglomeration of a fluorescent or phosphorescent species onto the bead and renders it (the bead) detectable and identifiable as a bead presenting a protein that participates in an interaction.

Beads decorated with an optically detectable species can be manually isolated, prior to identifying the interacting species, by merely picking then analyzing the beads that fluoresce.

Alternatively, the isolation of interacting species can be automated. Beads bearing fluorescent or phosphorescent moieties can be selected and isolated by utilizing a FACS (fluorescence-activated cell sorting) system, which is a technique well-known to those of ordinary skill in the art.

Interacting particle-attached species are first isolated. After isolating interacting species, the attached nucleic acid sequences are identified by sequencing, PCR, hybridization or a combination of these techniques. Alternatively, if the protein has been expressed off plasmid DNA, the encoding DNA can be released from the particles, and plated onto growth media. Once new colonies are grown, these provide a renewable source of the encoding DNA for analysis by any of the methods mentioned above (sequencing, PCR, hybridization or combinations of these techniques).

An advantage is that large numbers of proteins can be simultaneously tested to identify interacting pairs without apriori knowledge of the identity of either protein. Using standard techniques, libraries of complementary DNA (cDNA) can be inserted into protein expression plasmids or linear nucleic acid templates that have been modified to facilitate direct or indirect attachment to the encoded protein as described herein. For example, total mammalian cDNA can be inserted into plasmids that also contain sequences that encode a histidine tag and DNA binding sites for a yeast DNA binding protein, which would be presented on a particle that also presented NTA-Ni for the capture of the expressed protein. In this way, the expressed protein and its encoding DNA would be "attached" to each other via immobilization on a common particle. Similar genetic manipulations, when coupled with methods of the invention designed to detect and select interacting partners, enable the characterization of large numbers of interacting proteins and elucidation of interaction networks of entire genomes.

Methods are also described that can be used to functionally characterize an unknown protein of interest. With the recent sequencing of the human genome, it is now imperative to determine the function of newly identified genes. The number of genes in the human genome is estimated to be about 40,000. However, there are protein recognition motifs that are common to several proteins. One method of characterizing unknown genes is to determine which proteins the gene product interacts with. A less complex method is to determine which of the known protein recognition motifs the uncharacterized protein interacts with. A library of nanoparticles can be generated that each display a different protein recognition module, such as a kinase domain, phosphorylase, aPDZ domain, GRB1 and 2 domains, ERK, kringle, WW domains and the like. Uncharacterized proteins and their encoding DNA are separately attached to magnetic beads, then mixed with a library of colloid particles each bearing an interaction domain and its associated DNA. Interacting particles are then either optically or magnetically selected, then identified via analysis of attached nucleic acids or other encoding identifiers.

Fragments of DNA that encode proteins can be readily inserted into either plasmids or linear nucleic acid templates to facilitate expression of the encoded proteins. cDNA libraries that are inserted into a linear expression/translation template (for *in vitro* translation) offer certain advantages over insertion into plasmids. Hybrid nucleic acid expression templates can also be produced by generating the cDNA library with primers that contain both sequences that encode an affinity tag for the expressed protein and a functionality to facilitate attachment of both the gene product and its encoding DNA to a common surface. Linear expression templates can be readily modified with chemical functionalities to facilitate attachment to surfaces or functionalities to add a signaling capability. For example, protein encoding nucleic acid fragments can be genetically fused to nucleic acid strands that carry a biotin moiety. Biotinylated nucleic acids of any sequence are commercially available. Streptavidin has four binding sites for. Therefore, biotinylated nucleic acid expression templates bind to surfaces that present streptavidin.

Libraries of cDNAs that have been inserted into plasmids offer other advantages. Once an interaction has been detected, the interacting species and the plasmids that encode them can be isolated. The plasmids can then be introduced into a host cell in order to amplify the plasmid prior to sequencing or use in additional assays.

Another method involves fusing components of a cDNA library, for example representing the entire mammalian genome, to nucleic acids that encode a DNA-binding protein, or fragment thereof, and the DNA sequences to which it binds. Fusion proteins expressed from these templates will be able to bind to their encoding nucleic acids.

Also provided are methods for identifying affinity reagents for use in protein purification, immobilization and other general biochemical techniques. Because the interaction is not performed in a host cell, interacting components need not be entirely generated from genetic material. Preferably, a first set of chemical species is attached to recruitable beads along with a identifier that codes for the chemical identity of the immobilized chemical compound. A second set of genetically encoded species (preferable poly amino acids 4-14 amino acids in length) is attached to a linker species that has a signaling capability. The chemical and biological components, one or both of which may be attached to surfaces, are mixed together such that binding interactions can occur. Methods are used to select interacting components. The components of each interacting pair are then identified. This can be accomplished using methods such as sequencing attached nucleic acid identifiers or encoding plasmids or by using other analytical tools, which are better suited for the identification of chemical compounds and peptides. These methods include but are not limited to mass spec techniques and peptide sequencing techniques. Methods can also be used when the surface is the surface of a chip. With reference to Fig. 2, preferably, a set of derivatized colloids, each bearing a putative binding partner and identifiers, are introduced to a spatially addressable array chip that presents putative binding species in close proximity to an identifying oligonucleotide identifier. Chip-immobilized species are allowed to bind to colloid-immobilized species, then colloids bearing non-binders are washed away. A set of oligos (identifiers), which contains sequences complementary to all possible pair-wise combinations of the sequences that identify putative binding partners, is then incubated with the colloid-decorated chip. After rinsing, the oligos that have hybridized to the identifiers on the colloids and the chips represent identification of the putative binding species that interacted with each other. The "identifier" oligos are dissociated from the surface by any one of a number of methods including dissociation by heated water, chemical release, etc., and are then sequenced to reveal the identity of the interacting partners. The identifier oligos can also be enzymatically amplified at their specific locations while on the chip (such as by PCR) prior to the sequencing step. As shown, referring to Fig. 2, a chip 164 having a surface 166 includes a plurality of spatially-addressable regions 168, 170, etc. Each region includes a chemical or biological species (putative binding species) 174, 176, 178, etc. Each region also includes an oligonucleotide identifier 180, 182, 184, etc. Identifiers 180, 182, and 184 uniquely identify chemical or biological species 174, 176, and 178, respectively.

Following the reaction described above, as illustrated, one colloid particle 186 remains immobilized at surface 166. Colloid 186 includes, fastened thereto, a chemical or biological species 188 that binds to species 174, and oligonucleotide identifier 190. Binding of species 174 and 188 brings identifiers 180 and 190 into close proximity, whereby an interaction hybridization identifier 192 binds to the combination of identifiers 180 and 190. Identification of identifier 192 identifies the sequences of identifiers 180 and 190, identifying one or both of chemical or biological species 174 and 188.

Array chips that display a multitude of chemical or biological species that are putative binding partners, by and unique identifying oligos nearby, can be generated by a variety of techniques. One method involves forming heterologous self-assembled monolayers (SAMs) on surfaces that incorporate an entity that facilitates the attachment of a protein (e.g. a chelate/metal- terminated species that can participate in a SAM) and a second entity that facilitates the attachment of a nucleic acid species (e.g. a nucleic acid linker - terminated species that can participate in a SAM). For example, a mixed SAM can be formed from mixed thiol species that include a thiol-modified strand of DNA and a thiol terminated in nitrilo tri-acetic acid (NTA). NTA, when complexed with nickel, selectively captures histidine-tagged proteins. This heterologous SAM would then be able to capture any histidine-tagged protein and any strand of DNA that contains a sequence complementary to that displayed on the chip. Alternatively, a SAM exposing a single linking species (e.g. chelate/metal) can be formed, and used with both a binding partner that carries a polyamino acid tag and an oligonucleotide modified with a polyamino acid tag. The SAM-forming step is usually performed by incubated a gold-coated surface with various thiols in organic solvents. Organic solvents spread on metal surfaces. However, once the SAM has been formed, the steps of incubating a protein with the surface, then hybridizing a unique nucleic acid strand to the surface via a common "tail" that hybridizes to the surface-immobilized oligo, are both performed in aqueous buffer which beads up on SAM-coated surfaces. In this way, an entire surface may be coated with a universal SAM, which is then "dotted" with small volumes of protein and DNA to generate an array chip.

Alternatively, various species of DNA identifiers that are able to bind to a surface, for example via thiol modification, in aqueous solution, can be dotted onto an underivatized gold-coated surface. After some incubation period, the chip is then exposed to a solvent containing "filler" thiols. These filler species may also contain a thiol species that facilitates the attachment of proteins to the surface, which may be carried out in a spatially addressable way.

Standard gene chips can also be used with the methods described herein, if they are modified to allow the placement of proteins in close proximity to an identifier.

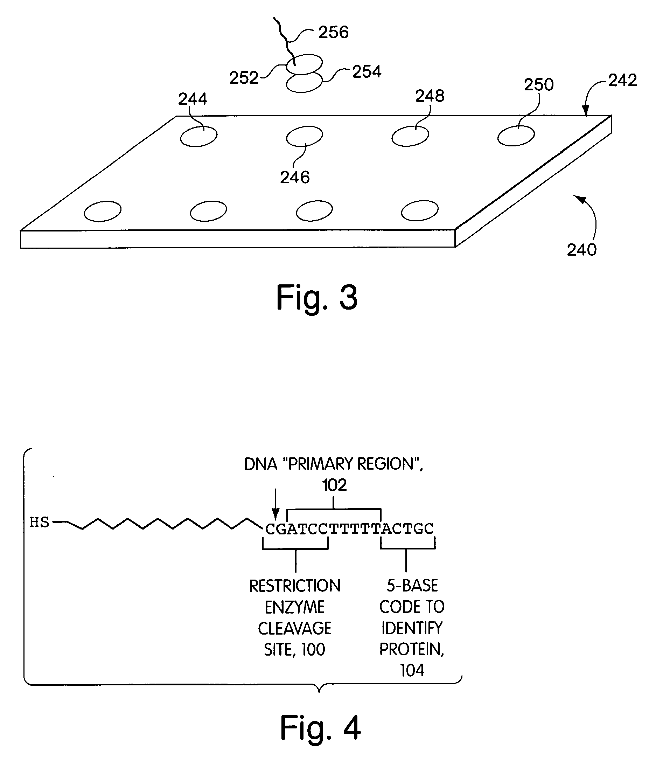

Referring now to Fig. 3, a
chip 240 includes a surface 242 to which a plurality of chemical or biological species 244 - 250, etc., are fastened. Exposure of the surface to colloid particles 252 each carrying a chemical or biological species 254 and an oligonucleotide identifier 256 results in binding between species 246 and 254, as illustrated. Subsequently, localized cleavage and identification of identifier 256, or localized PCR or hybridization can identify binding. Species 244 - 250 can be identical, with non-identical species attached to colloids, or species 244 - 250 can be different with identical species attached to colloids exposed to the surface, or both species attached to the surface and the species attached to the colloids can be varied.

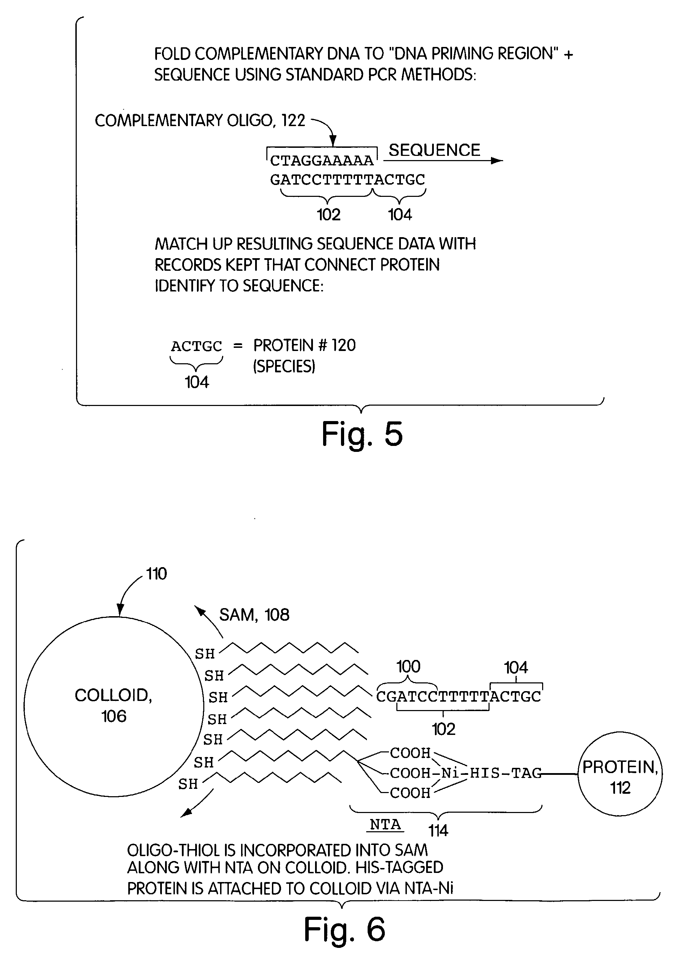

Referring now to Fig. 4, one arrangement for fastening an oligonucleotide to a surface is illustrated. Fig. 4 illustrates a self-assembled monolayer-forming species incorporating an oligonucleotide identifier. Specifically, the self-assembled monolayer-forming species is a long-chain alkyl thiol including a restriction enzyme cleavage site 100, a DNA priming region 102 linked to the cleavage site, and a 5-base oligonucleotide identifier 104 linked to the priming region. It is to be understood that the priming region can be defined by any entity that can link the oligonucleotide identifier to the self-assembled monolayer-forming species and also promote sequencing as will be described below with reference to Fig. 5. It is also to be understood that the arrangement of Fig. 4 is by example only, and other techniques for linking an oligonucleotide identifier to a surface can be used.

Fig. 6 illustrates incorporation of the species of Fig. 4 into a self-assembled monolayer onto a surface, specifically a surface of a colloid, along with immobilization of a biological binding partner to the same surface. A colloid particle 106 has a gold surface to which thiols will bind and upon which thiol-contained self-assembled monolayer-forming species will form self-assembled monolayers. A self-assembled monolayer 108 is formed on a surface 110 of colloid 106, including both the species of Fig. 4 and an immobilized biological binding partner (protein) 112. As illustrated, species 112 is fastened to a self-assembled monolayer-forming species that is incorporated into SAM 108. Specifically, the species is linked to the self-assembled monolayer-forming species via a metal binding tag/metal/chelate linkage 114.

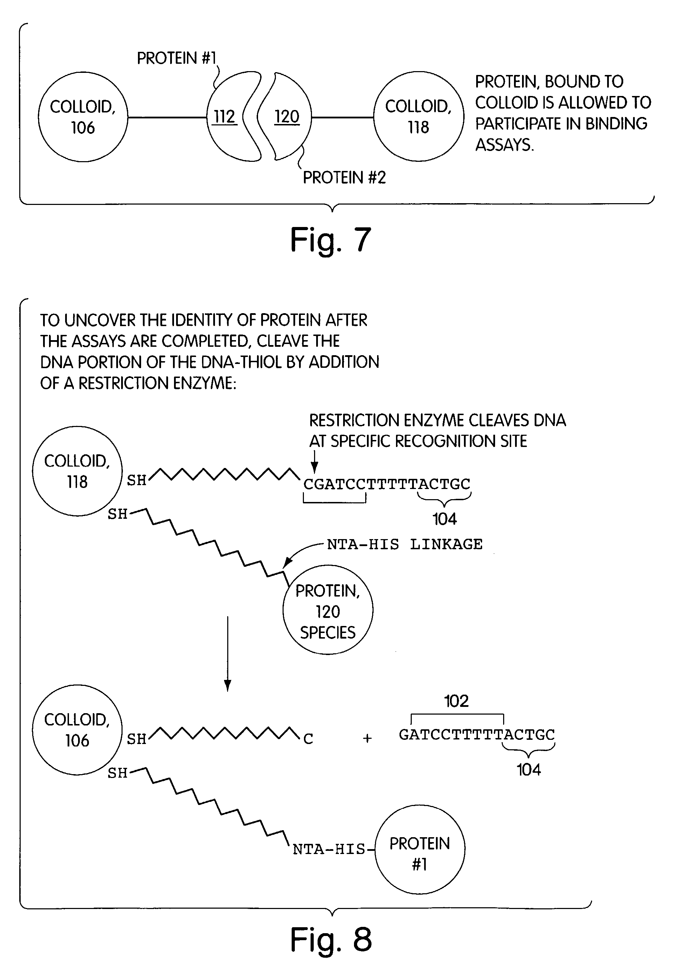

Fig. 7 illustrates, schematically, colloid particle 106 carrying binding species 112 and a second colloid 118 carrying a chemical or biological species 120 which is a biological binding partner of species 112. As illustrated, species 112 and 120 are each protein, although as would be understood by those of ordinary skill in the art, other binding species can be used. For example, any of a variety of proteins, peptides, or other species can define species 112 and 120, and species 112 and 120 can be fastened to the colloid particle via other affinity tags. The oligonucleotide identifier and/or chemical or biological species also can be fastened to the surface of the colloid via any of a variety of affinity tags, or a carboxylic acid thiol via EDC/NHS coupling. Alternatively, a species of interest can be attached to a thiol for direct attachment to the colloid.

Self-assembled monolayer-forming species, as illustrated, can include long carbon chains such as 11 carbons or greater.

Once species 112 and 120 are allowed to participate in a binding assay (including any assay described in the documents incorporated herein by reference such as bead coloration assays, colloid-colloid assays, etc.), if one of species 112 or 120 is identified as a binding partner in the assays, the identity of which would be desirably known, then the identity of the species can be uncovered as follows. For purposes of this discussion it is assumed that colloid 106 carries a known binding partner 112 and colloid 118 carries a species 120 that was not known to be a biological binding partner of species 112. Thus, once identification of the binding between species 112 and 120 is known (for example via aggregation of colloid particles), where a variety of other colloid particles carrying species other than 120 were involved in the assays and may have bound to species 112, then the identity of species 120 is desirably determined. With reference to Fig. 8, a restriction enzyme is added to cleave oligonucleotide identifier 104 from colloid particle 118 (along with the priming region 102). Subsequently, with reference to Fig. 5 an oligonucleotide primer 122 complementary to the priming region 102 is added, and normal PCR-based sequencing, or other standard sequencing, is performed on the cleaved oligonucleotide to decipher the sequence of the oligonucleotide identifier. Standard fluorescent sequencing can be carried out, for example. Once the sequence of the oligonucleotide identifier 104 is identified, it in turn identifies species 120 as that species that had been bound to colloid particle 118 to which identifier 104 also had been bound; identifier 104 had been correlated to species 120 prior to running of the assay.

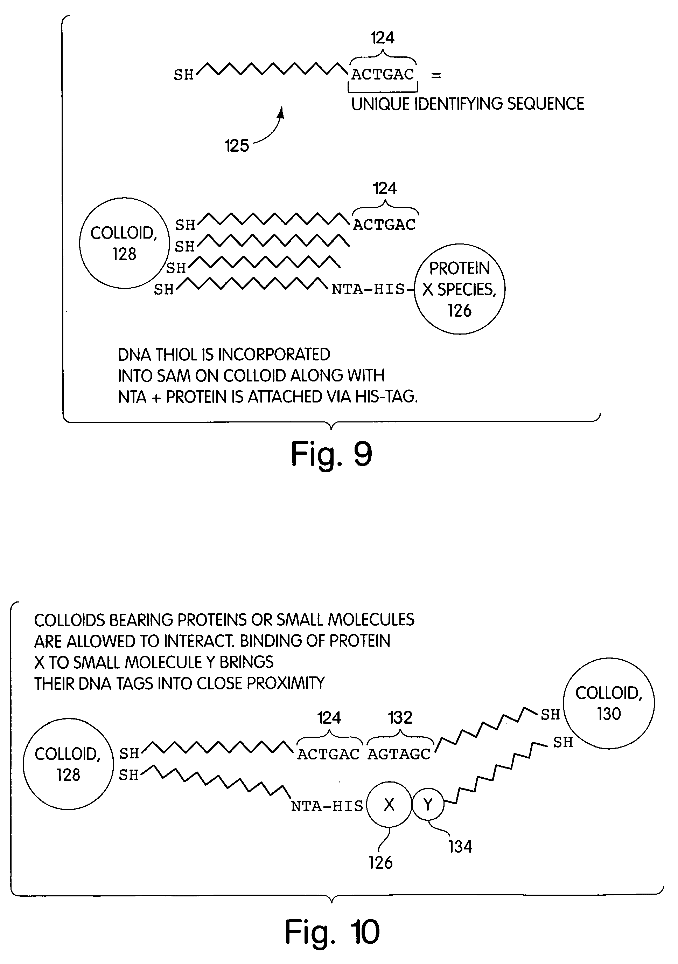

Another technique for use of oligonucleotide identifiers is described now with reference to Figs. 9-15. Fig. 9 illustrates an oligonucleotide identifier 124 which is part of a self-assembled monolayer-forming species 125. Identifier 124 can be fastened to and form a part of SAM-forming species 125 via any technique, and need not include a restriction enzyme cleavage site or priming region 100 and 102, respectively, as illustrated in the figures above. Fig. 9 also illustrates identifier 124 and a chemical or biological species 126, each fastened to the surface of a colloid particle 128 via self-assembled monolayer-forming species. That is, a SAM on the surface of colloid 128 includes both identifier 124 and species 126. Identifier 124 and species 126 can be fastened to colloid 128 in any manner known to those of ordinary skill in the art, including any manner described herein. Referring now to Fig. 10, colloid 128 is brought into proximity with a second colloid particle 130, which carries its own oligonucleotide identifier 132 and its own chemical or biological species 134. If chemical or biological species 126 and 134 bind, for example if they are biological binding partners, then oligonucleotide identifiers 124 and 132 will be brought into close proximity with each other. As an example of an assay shown in Fig. 10 a plurality of colloids 128 each carrying a plurality of binding partners 126 is provided and mixed with a plurality of colloids 130, each carrying a different species that may or may not be a binding partner of species 126. Colloid 130 carries species 134 which is a binding partner of species 126, as determined by the aggregation of colloid particles 128 with colloid particles 130, for example. The binding of species 126 and 134 having been determined, it is desirable (in this example) to determine the identity of species 134.

Alternatively, a large number of colloid particles carrying a wide variety of potential binding partners can be admixed, where a variety of different binding interactions may occur.

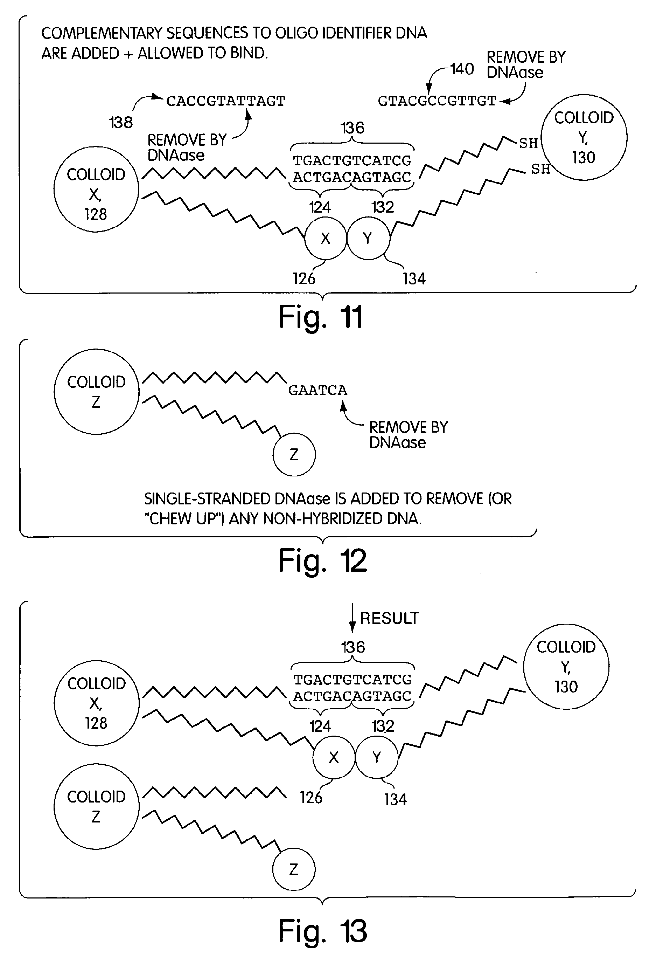

In any event, once binding between species 126 and 134 has occurred (e.g. via detection of a color change from pink to blue as aggregation of colloid particles occurs), then the identity of species 126, or 134, or both is determined. Referring now to Fig. 11, identifiers 124 and 132 together define a sequence that may be complementary to an interaction hybridization identifier 136. Where this is the case, identifier 136 will bind to the combination of identifiers 124 and 132. This will occur when a variety of interaction hybridization identifiers are added to the assay, each corresponding to a different potential combination of oligonucleotide identifiers fastened to colloid particles. If only two species such as proteins or small molecules are being assayed for binding, then only one complementary sequence would need to be added.

Subsequently, all non-bound oligonucleotide is de-activated, e.g. by adding a DNAase that degrades single-stranded DNA (Fig. 12). This eliminates any oligonucleotide identifiers present on non-interacting colloids that did not participate in a binding event, and any auxiliary, non-bound interaction hybridization identifiers (138 and 140). Fig. 13 illustrates the result of this step.

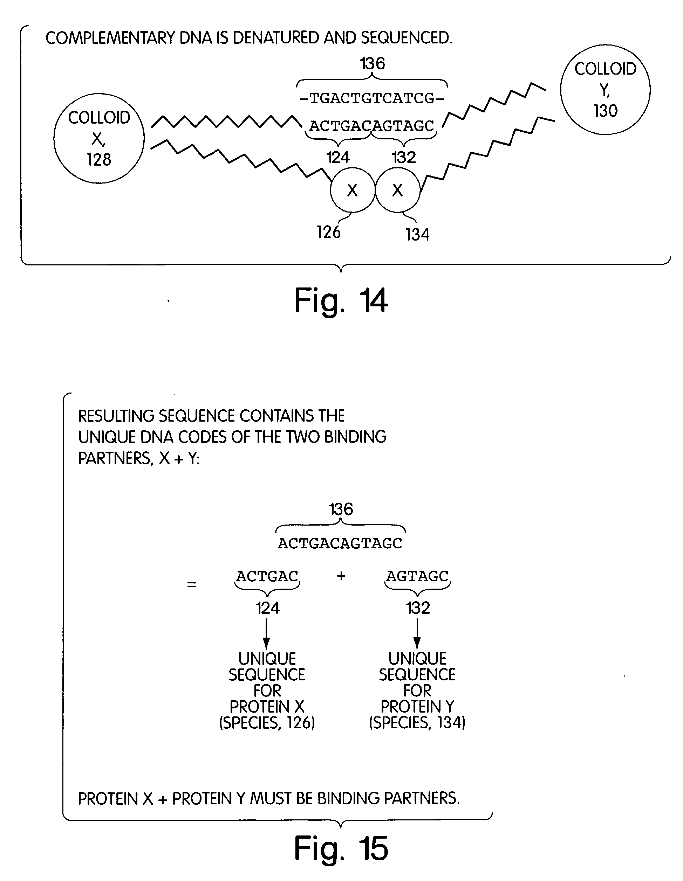

With reference to Fig. 14, interaction hybridization identifier 136 is removed from and isolated relative to identifiers 124 and 132 via, for example, denaturization (by boiling or addition of salt or Triton solution, etc.). Then, identifier 136 is sequenced (Fig. 15). With the identification of species 136, the identity of identifiers 124 and 132 is determined, and thereby the identity of one or both of species 126 and 134 can be determined (identifier 124 had been correlated to species 126 and identifier 132 had been correlated to species 134 prior to running of the assay).

### Example: Demonstration of Control of SAM permeability to electrons

This example demonstrates the ability to form a SAM including enhanced electronic communication. The SAM is formed on a surface that includes a mixture of a first, tight-packing species and a second species of different molecular structure that enhances the permeability of the SAM to electronic communication. A defect, or opening, is formed in the SAM allowing fluid to which the surface is exposed to communicate electrically with the surface. Specifically, certain small sulfur containing compounds having disruptive structures relative to the SAM as a whole were stably incorporated into a SAM, and greater permeability to electrons was demonstrated. This example demonstrates that a surface can be made electrically relatively conductive, and then support cell growth.

A water-soluble ferrocene derivative was dissolved in the electrolyte solution: 100mM solution of ferrocenedicarboxylic acid in 500uM NaClO₄. The working electrode was a gold-coated electrode derivatized with a SAM comprised of varying amounts of 2-unit molecular wire (MFI), The height of the peak at a characteristic ferrocene potential was plotted as a function of molecular wire density. As a negative control, a gold-coated electrode was derivatized with an insulating SAM comprised of 100% tri-ethylene glycol terminated thiol. This system was used to test the conductivity of electrodes modified with a panel of sulfur-containing compounds. The compounds were dissolved in DMF at 50% candidate compound and 50% tri-ethylene glycol terminated thiol. Electrodes were derivatized as described in Example 1. SAMs were formed on gold chips from 500 micromolar triethylene glycol-terminated thiol and 500 micromolar of either mercaptobenzothiazole or 2-mercaptoethyl ether in DMF. The chips were clamped between a flat substrate and a 1 ml capacity silicon gasket. A solution of ferrocene dicarboxyllic acid was dissolved in 500 micro molar NaClO4 and placed in the silicon gasket with a Ag/AgCl reference electrode and a Pt auxiliary electrode. The gold chip was connected as the working electrode. The system was analyzed by ACV. The magnitude of the current peaks, resulting from the ferrocene in solution communicating with the electrode, was an indicator of the ability of the trial compounds to make the SAM more permeable to electron flow by creating defects within the SAM.

### Example: Detection of Protein-protein interactions

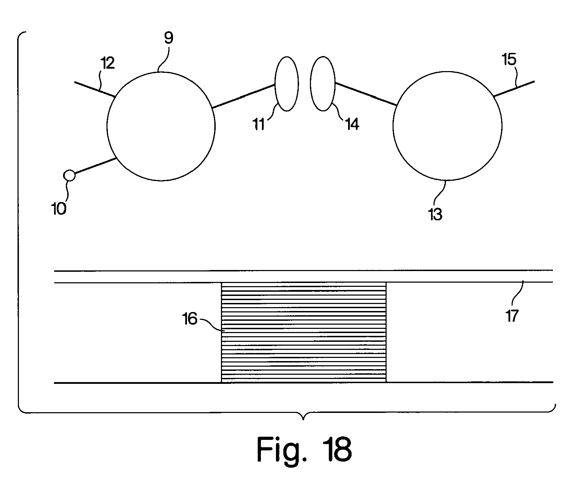

This example demonstrates the utility of a colloid particle having an immobilized signaling entity and an immobilized protein. (See Fig. 18)

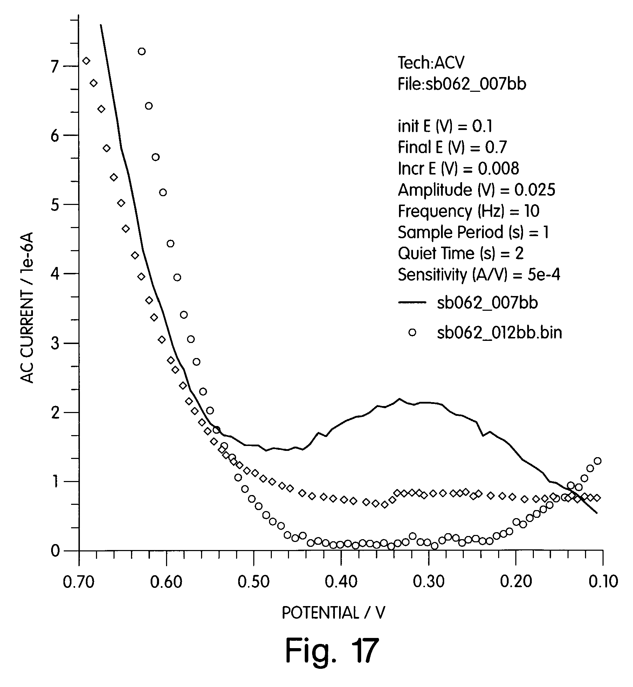

Histidine-tagged Glutathione-S-Transferase (GST-His) was attached to NTA-SAM-coated colloids, displaying 40uM NTA-Ni and 100uM ferrocene-thiol. Commercially available magnetic beads presenting protein A were coated at 1/10 binding capacity with anti-GST antibody, added at a 1:5 ratio to the GST-colloids, and measured on a 50% MF-1 SAM-coated electrode, which was placed on top of a magnet. The magnet pulled the magnetic beads onto the electrode surface to form a thick, visible precipitate. The GST-colloids were brought down to the electrode surface by the interaction with the GST-antibody on the magnetic beads to give a current peak at approximately 280mV. Two negative controls were run, one where GST was not attached to the colloid surface, and another where the GST antibody was not attached to the magnetic beads. Neither negative control gave a current peak. Fig. 17 plots the results of this demonstration. Solid line represents interaction between GST-His-presenting colloids and anti-GST/Ab on magnetic beads. Open circles represent magnetic beads presenting the antibody incubated with colloids that did not present GST. Closed circles represent beads not presenting the antibody, incubated with colloids that presented GST.

### Prophetic Example: Massively Parallel Analysis of Protein-Protein Interactions: Elucidating the Interaction Map of the Human Proteome.

The following prophetic example describes how to perform massively parallel analysis of protein-protein interactions, which is particularly useful when proteins are as yet uncharacterized. Here, this method is used to elucidate the protein interaction map of the human proteome. A subset of, or the entire set of, proteins of the proteome is expressed with affinity tags to facilitate attachment of the expressed protein to sets of particles. Particle-immobilized proteins are pooled together and allowed to interact. Interacting pairs are selected from the pooled mixture by a reiterative magnetic selection/dilution process. Following the selection/dilution process, the identity of interacting partners is determined. The selection step reduces the complexity of the problem by eliminating the need to analyze non-interacting proteins.

A diagram illustrating how two binding partners can be detected through magnetic recruitment is provided in Fig. 18. As shown, nanoparticle 9 is attached to electroactive signaling entity 10. An immobilized chemical or biochemical species 11 is also immobilized to the nanoparticle 9, as is oligo identifier 12 which is thereby associated with species 11. A second chemical or biochemical species 14 is immobilized on magnetic bead 13. Second oligo identifier 15 is also immobilized with respect to magnetic bead 13 and thus is associated with second species 14. As binding occurs between species 11 and 14 (as shown in Fig. 18), a single hetero-particle forms that can then be magnetically drawn to sensing electrode 17 by electromagnet 16. Detection may be facilitated by signaling entity 10.

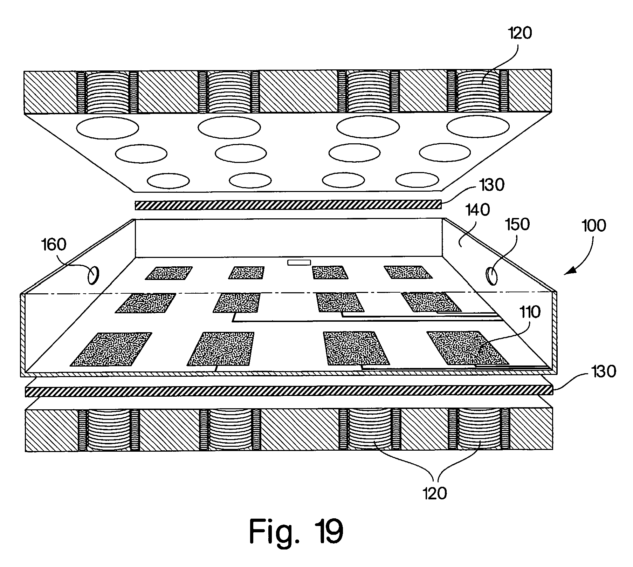

In a more specific example, proteins and their encoding DNA molecules are indirectly connected to each other by co-immobilizing both on a common particle or bead, wherein each particle (or bead) presents a single protein species and its encoding DNA. Connecting the expressed protein to its encoding DNA expedites the identification of each set of interacting proteins after the selection/dilution process. Each protein and its encoding DNA are immobilized on two different kinds of particles: a recruitable particle and a signaling particle. The sizes of the particles are also different such that smaller signaling particles can form satellites around each larger recruitable particle. In this example, each protein and its encoding DNA are immobilized on a single 4-10 micron magnetic bead as well as on a multitude of fluid suspendable nanoparticles, that are 4-40 nm in diameter and bear electroactive signaling entities. When a first species on a magnetic bead biologically interacts with a second species on a signaling nanoparticle, the recruitable particle becomes "connected" to the signaling particle. These hetero-particle-complexes are then magnetically recruited to a sensing electrode (see Fig. 19), where they can deliver a signal, such as an electronic or electrochemical signal. To facilitate multiplexed analysis, the particles are magnetically attracted to an electrode array that has a number of individually addressable electrode pads. Beneath each electrode pad is an individually controllable electromagnet, such that the magnetic field above each pad can be selectively turned off and on. However, magnetic particles that are not connected to signaling particles, which cannot deliver a signal, may also be recruited to the same electrode pad that delivers a positive signal. These non-signaling magnetic beads are electromagnetically released from the pad and washed out of the interaction reservoir. Signaling nanoparticles that do not interact with species on magnetic particles remain in a homogeneous suspension and do not deliver a signal but are also washed out of the interaction reservoir. Both of these purging functions are accomplished by maintaining the magnetic fields beneath electrode pads that deliver a positive signal, to retain interacting complexes, while the magnetic fields beneath pads that do not deliver a positive signal are driven to zero to release non-interacting magnetic beads. A port 150 (Fig. 19) is opened and fluid is washed out of the interaction reservoir carrying away non-interacting magnetic beads and non-interacting nanoparticles. The port is closed, all magnetic fields are driven to zero and more buffer is added through a second port 160 along with mechanical agitation to resuspend and redistribute the particles. All the magnetic fields beneath all the electrode pads are turned on again and the selection/dilution process is repeated until, statistically, each pad that delivers a positive signal contains a single magnetic bead bound to a multitude of signaling nanoparticles.

To determine the identity of interacting proteins, an array of magnetic pins, whose dimensions correspond to that of the electrode array is juxtaposed over the electrode array and the magnetic fields beneath the entire electrode array are driven to zero such that each magnetic pin captures a single hetero-particle complex. The loaded pin array is dipped into a multi-well plate, of compatible dimensions, each well of which is filled with solution containing DNA amplification reagents. The respective encoding DNA sequences (immobilized on the nanoparticles and the bead) are amplified by PCR or similar technique and sequenced to reveal the identity of each set of interacting proteins.

### Prophetic Example: Preparation of Proteins for co-immobilization on surfaces that also present coding identifiers.

A DNA sequence that encodes each protein member of the proteome is inserted into a bacterial protein expression vector. The expression vector carries an affinity tag, (His)₆ in this case, tandem repeats of Gal4 consensus sequences, and 2 sequences that flank the protein identification sequence, to which PCR primers can bind. The histidine-tag facilitates the attachment of the expressed protein to the particle. The Gal4 consensus sequences act to tether the encoding DNA to the particle via the interaction between the recognition motif and a particle-immobilized yeast DNA binding domain, which in this case is a GST-Gal4 fusion protein. The DNA binding domain of Gal4 (aa' 1-100) binds to the consensus sequence CGGattAgAagcCgCCGAG and the GST binds to a glutathione moiety on the particle. Proteins are separately expressed in a cell-free translation system to reduce the abundance of irrelevant proteins and cell debris. Following protein expression, each expression mixture contains the encoding DNA and the expressed protein. Each protein expression mixture is divided into 2 aliquots. A single magnetic bead (4-10 microns in diameter) is added to a first aliquot and a quantity of NTA-glutathione-SAM-coated nanoparticles is added to a second aliquot. Particles are pelleted and washed to remove protein that is not particle-bound. Particles and beads are pooled together in subsets of 1000 species per pool and subjected to magnetic selection/dilution and electrochemical analysis. In this manner, unidentified proteins can be bound to the same bead or particle that also binds the corresponding encoding DNA.

In examples were colloid particles (nanoparticles) are used, they can be prepared as described in the above-referenced international patent publication nos. WO 00/43791 and WO 00/043783.

### Prophetic example: Electrochemical Analysis

An electrochemical analyzer from CH Instruments (Austin, TX) is used to detect interactions between species immobilized on magnetic beads and species immobilized on colloids that also bear redox active metals. The instrument is modified to facilitate multiplexed detection. In this case, the redox active metals are ferrocene derivatives. Pads of the electrode array are individually addressable and act as the working electrode. In this case, the pads are gold-coated and derivatized with conductive self-assembled monolayers. A Ag vs. Ag/Cl reference electrode is used with a Pt auxiliary. Electrodes are scanned using Alternating Current Voltammetry (ACV) with a 25mV overpotential at a frequency of 10 Hz..

### Prophetic Example: Design of Electrode Array Sandwiched between Individually addressable Electromagnets

Electrode arrays 100 (Fig. 19) having 300-500 electrode pads 110 are constructed by plating gold over Ni⁺⁺. Electrode pads are 50-500 microns on edge and are sandwiched between sets of individually addressable Helmholtz electromagnets 120 such that a magnetic field gradient can be generated to recruit, then hold magnetic beads at the pad surface (see Fig. 19). The direction of the current is reversed to drive the magnetic field to zero when it is desirable to release magnetic beads from the surface to wash away or redistribute. To ensure that the interaction reservoir is thermally isolated from the electromagnet arrays so that heat does not denature proteins in solution, a layer of insulative material 130 is placed between the electromagnets and the interaction reservoir 140.

### Prophetic Example: Calculation of Protein Sets and Electrode Pad Number

To generate the protein interaction map of the entire proteome, one needs to divide the proteome into subsets, which are then tested for interaction with every other subset. Assuming that there are about 50,000 proteins of interest, the proteome is divided into 50 sets of 1000 proteins each. Each group of 1000 proteins is then tested for interaction with every other group of 1000, resulting in 50X50 matrix or 2500 separate experiments. The number of proteins in each subset determines the number of electrode pads in each array. If we assume that each protein has a single binding partner, then each protein has a 1/50 chance of finding that partner when tested for interaction with one of the 50 subsets of proteins. However, each protein probably has on average 5 relevant binding partners, increasing the probability of finding a binding partner within a subset to 1/10. That means that for 2000 proteins in one pooled interaction mix, 200 will deliver a positive signal, which implies that the electrode array should have 300-500 pads. Low-level signals from non-specific binding events are minimal because of competitive inhibition by relevant binders. However, the occurrence of false positives is minimized when a signal threshold is set, wherein signals below the threshold are counted as negatives. Relative affinities are determined by comparison of the degree of interaction of a first binding species and a second binding species with a third target protein to which the first and second bind.

### Prophetic Example: Determining the Binding Partners of a Single Target Protein with a Large Pool of Candidate Binding Partners

This prophetic example describes how to identify proteins, from a large pool of putative binding partners, which interact with a single target protein. Proteins from the large pool are prepared as described above in Example 30, and immobilized only on signaling nanoparticles. The target protein is immobilized on a set of magnetic beads. Because the identity of the target protein is known, it is not necessary to co-immobilize its encoding DNA. Interacting partners are selected by electrochemical analysis as described above.

### Prophetic Example: Selection of Interacting Protein Partners by FACS Analysis

This example describes how to identify the binding partners of a single target protein. The target protein is immobilized on a set of beads that are 4-25 microns in diameter. Putative binding partners, which may be prepared as described above or generated from a cDNA library, are co-immobilized along with their encoding DNA onto nanoparticles that bear fluorescent signaling moieties. When a bead-immobilized protein interacts with a species immobilized on nanoparticles, the bead becomes decorated with fluorescent nanoparticles and can be isolated by FACS (fluorescent activated cell sorting) analysis after which the attached DNA of each interacting species is sequenced to identify the binding partners.

## Claims

1. A method for simultaneous screening for interactions between chemical or biological species, comprising:
providing a first chemical or biological species, immobilized relative to a first surface, and a first oligonucleotide identifier independently associated with the first surface;
providing a second chemical or biological species, immobilized relative to a second surface;
allowing the first species to participate in a chemical or biological interaction with the second species;
determining participation of the first and second species in the interaction; and
determining the identity of the first oligonucleotide identifier, thereby identifying the first species;
wherein the first surface is the surface of a nanoparticle.

2. A method as claimed in claim 1, wherein the nanoparticle is a colloid particle.

3. A method as claimed in claim 2, wherein the nanoparticle is a gold colloid particle.

4. A method as claimed in any of the preceding claims, wherein the second surface is the surface of a recruitable particle, magnetic bead, colloid particle or chip.

5. A method as claimed in claim 4, wherein the second surface is the surface of a bead.

6. A method as claimed in any of the preceding claims, wherein the first oligonucleotide identifier encodes the first species.

7. A method as claimed in claim 6, wherein the first species is a protein.

8. A method as claimed in any of the preceding claims, wherein the nanoparticle is a colloid particle, and the first species and first oligonucleotide identifier are immobilized relative to the surface of the colloid particle via a self-assembled monolayer.

9. A method as claimed in claim 8, wherein the first species is fastened to the surface of the colloid particle via a metal binding tag/metal/chelate linkage.

10. A method as claimed in claim 8 or 9, wherein the first oligonucleotide identifier comprises an oligonucleotide species including a linker portion that is the complement of an oligonucleotide linker on the surface of the colloid particle, and the first oligonucleotide identifier is fastened to the surface of the colloid particle via the oligonucleotide linker.

11. A method as claimed in any of the preceding claims, wherein the first oligonucleotide identifier is immobilized to the first surface via a nucleic acid binding protein, preferably via a DNA binding protein.

12. A method as claimed in any of claims 1 to 10, wherein the first oligonucleotide identifier is modified to facilitate attachment to the first surface via a recognition protein.

13. A method as claimed in any of claims 1 to 10, wherein the first oligonucleotide identifier is biotinylated to facilitate attachment to the first surface via streptavidin.

14. A method as claimed in any of the preceding claims, wherein the first oligonucleotide identifier comprises plasmid DNA, a protein expression vector or linear DNA.

15. A method as claimed in any of the preceding claims, wherein the first oligonucleotide identifier comprises a protein expression template and/or a product of a polymerase chain reaction.

16. A method as claimed in any of the preceding claims, wherein a signaling entity is immobilized relative to at least one of the first oligonucleotide identifier and first species.

17. A method as claimed in any of claims 1 to 16, wherein the first oligonucleotide identifier is modified to include a signaling entity.

18. A method as claimed in claim 17, wherein the first oligonucleotide identifier is generated via PCR using primers modified with signaling entities.

19. A method as claimed in any of claims 16 to 18, wherein the sequence of the first oligonucleotide identifier is determined via fluorescent sequencing.

20. A method as claimed in any of the preceding claims, wherein a second oligonucleotide identifier is independently associated with the second surface, comprising determining the sequence of the first and second oligonucleotide identifiers.

21. A method as claimed in claim 20, comprising:
allowing the first and second species to participate in the interaction, thereby immobilizing the first and second colloid particles relative to each other and bringing the first oligonucleotide identifier into proximity with the second oligonucleotide identifier;
exposing the first and second oligonucleotide identifiers to an interaction hybridization identifier, which is complementary to the combination of the first and second oligonucleotide identifiers, and allowing the interaction hybridization identifier to bind to the first and second oligonucleotide identifiers; and
identifying the interaction hybridization identifier, thereby identifying the first and second oligonucleotide identifiers, and thereby identifying the interaction.

22. A method as claimed in claim 21, comprising, prior to the identifying step, de-activating any non-hybridized oligonucleotide.

23. A kit comprising a plurality of gold nanoparticles,
wherein the surface of each nanoparticle is completely covered by a self assembled monolayer that incorporates a carboxylate group, a metal chelate such as nitrilotriacetic acid, biotin, n-hydroxy-succinimide, glutathione, streptavidin or fragment thereof that binds only one biotin and an oligonucleotide.

24. A kit as claimed in claim 23, wherein the nanoparticle bears a carboxylate group, a metal chelate such as nitrilotriacetic acid, biotin, n-hydroxy-succinimide, glutathione, streptavidin or fragment thereof that binds only one biotin, so as to bind the protein or peptide to the nanoparticle.

25. A kit as claimed in claim 23 or claim 24, further comprising a plurality of surfaces each of which bears or is adapted to bear a protein or peptide;
wherein each surface specifically binds or is adapted to specifically bind the protein or peptide.

26. A kit comprising a plurality of gold nanoparticles,
wherein the surface of each nanoparticle is completely covered by a self assembled monolayer that incorporates thiols terminated with a binding partner of an affinity tag, and an oligonucleotide.

27. A kit as claimed in claim 26, wherein the affinity tag is a metal binding tag and the binding partner of the affinity tag is a species terminating in a chelate coordinating a metal, or wherein the affinity tag is GST and the binding partner of the affinity tag is glutathione, or wherein the affinity tag is streptavidin and the binding partner of the affinity tag is biotin.

28. A kit as claimed in claim 27, wherein the affinity tag is a histidine tag and the binding partner of the affinity tag is NTA-nickel.

29. A kit as claimed in any of claims 23 to 28, wherein the oligonucleotide encodes the protein or peptide.

30. A kit as claimed in any of claims 23 to 29, wherein the nanoparticles each bear a protein or peptide.

31. A kit as claimed in claim 30, wherein the protein or peptide is an antibody or antigen.

## Patentansprüche

1. Verfahren zum gleichzeitigen Prüfen auf Interaktionen zwischen chemischen oder biologischen Stoffen, beinhaltend:
Bereitstellen eines ersten chemischen oder biologischen Stoffes, immobilisiert relativ zu einer ersten Oberfläche, und eines ersten Oligonukleotid-Identifikators, unabhängig mit der ersten Oberfläche assoziiert;
Bereitstellen eines zweiten chemischen oder biologischen Stoffes, immobilisiert relativ zu einer zweiten Oberfläche;
Erlauben des ersten Stoffes, an einer chemischen oder biologischen Interaktion mit dem zweiten Stoff teilzunehmen;
Bestimmen der Teilnahme des ersten und zweiten Stoffes an der Interaktion; und
Bestimmen der Identität des ersten Oligonukleotid-Identifikators, wodurch die ersten Stoffe identifiziert werden;
wobei die erste Oberfläche die Oberfläche eines Nanopartikels ist.

2. Verfahren gemäß Anspruch 1, wobei das Nanopartikel ein Kolloidpartikel ist.

3. Verfahren gemäß Anspruch 2, wobei das Nanopartikel ein Goldkolloidpartikel ist.

4. Verfahren gemäß einem der vorhergehenden Ansprüche, wobei die zweite Oberfläche die Oberfläche eines rekrutierbaren Partikels, magnetischen Kügelchens, Kolloidpartikels oder Chips ist.

5. Verfahren gemäß Anspruch 4, wobei die zweite Oberfläche die Oberfläche eines Kügelchens ist.

6. Verfahren gemäß einem der vorhergehenden Ansprüche, wobei der erste Oligonukleotid-Identifikator den ersten Stoff codiert.

7. Verfahren gemäß Anspruch 6, wobei der erste Stoff ein Protein ist.

8. Verfahren gemäß einem der vorhergehenden Ansprüche, wobei das Nanopartikel ein Kolloidpartikel ist und der erste Stoff und der erste Oligonukleotid-Identifikator relativ zur Oberfläche des Kolloidpartikels über eine selbst organisierte Monoschicht immobilisiert sind.

9. Verfahren gemäß Anspruch 8, wobei der erste Stoff an der Oberfläche des Kolloidpartikels über eine Metallbindungs-Tag-/Metall-/Chelat-Verbindung befestigt ist.

10. Verfahren gemäß Anspruch 8 oder 9, wobei der erste Oligonukleotid-Identifikator einen Oligonukleotid-Stoff beinhaltet, einschließlich einem Linker-Teil, der die Ergänzung eines Oligonukleotid-Linkers auf der Oberfläche des Kolloidpartikels ist, und der erste Oligonukleotid-Identifikator an der Oberfläche des Kolloidpartikels über einen Oligonukleotid-Linker befestigt ist.

11. Verfahren gemäß einem der vorhergehenden Ansprüche, wobei der erste Oligonukleotid-Identifikator an der ersten Oberfläche über ein nukleinsäurebindendes Protein, vorzugsweise über ein DNA-bindendes Protein, immobilisiert ist.

12. Verfahren gemäß einem der Ansprüche 1 bis 10, wobei der erste Oligonukleotid-Identifikator modifiziert ist, um die Anbringung an der ersten Oberfläche über ein Erkennungsprotein zu erleichtern.

13. Verfahren gemäß einem der Ansprüche 1 bis 10, wobei der erste Oligonukleotid-Identifikator biotinyliert ist, um die Anbringung an der ersten Oberfläche über Streptavidin zu erleichtern.

14. Verfahren gemäß einem der vorhergehenden Ansprüche, wobei der erste Oligonukleotid-Identifikator Plasmid-DNA, einen Proteinexpressionsvektor oder Linear-DNA beinhaltet.

15. Verfahren gemäß einem der vorhergehenden Ansprüche, wobei der erste Oligonukleotid-Identifikator eine Proteinexpressionstemplate und/oder ein Produkt einer Polymerase-Kettenreaktion beinhaltet.

16. Verfahren gemäß einem der vorhergehenden Ansprüche, wobei eine Signalisierungseinheit relativ zu mindestens einem des ersten Oligonukleotid-Identifikators und ersten Stoffes immobilisiert ist.

17. Verfahren gemäß einem der Ansprüche 1 bis 16, wobei der erste Oligonukleotid-Identifikator modifiziert ist, um eine Signalisierungseinheit einzuschließen.

18. Verfahren gemäß Anspruch 17, wobei der erste Oligonukleotid-Identifikator über PCR mittels Primer, modifiziert mit Signalisierungseinheiten, generiert wird.

19. Verfahren gemäß einem der Ansprüche 16 bis 18, wobei die Sequenz des ersten Oligonukleotid-Identifikators über Fluoreszenzsequenzierung bestimmt wird.

20. Verfahren gemäß einem der vorhergehenden Ansprüche, wobei ein zweiter Oligonukleotid-Identifikator unabhängig mit der zweiten Oberfläche assoziiert ist, beinhaltend das Bestimmen der Sequenz des ersten und zweiten Oligonukleotid-Identifikators.

21. Verfahren gemäß Anspruch 20, beinhaltend:
Erlauben des ersten und zweiten Stoffes, an der Interaktion teilzunehmen, wodurch die ersten und zweiten Kolloidpartikel relativ zueinander immobilisiert werden und der erste Oligonukleotid-Identifikator in die Nähe zum zweiten Oligonukleotid-Identifikator gebracht wird;
Exponieren des ersten und zweiten Oligonukleotid-Identifikators gegenüber einem Interaktionshybridisierungsidentifikator, der ergänzend zur Kombination des ersten und zweiten Oligonukleotid-Identifikators ist, und Erlauben des Interaktionshybridisierungsidentifikators, sich am ersten und zweiten Oligonukleotid-Identifikator zu binden; und
Identifizieren des Interaktionshybridisierungsidentifikators, wodurch der erste und zweite Oligonukleotid-Identifikator identifiziert werden und wodurch die Interaktion identifiziert wird.

22. Verfahren gemäß Anspruch 21, beinhaltend, vor dem Identifizierungsschritt, Deaktivieren von nicht hybridisiertem Oligonukleotid.

23. Kit, beinhaltend eine Vielzahl von Goldnanopartikeln,
wobei die Oberfläche von jedem Nanopartikel vollständig mit einer selbst organisierten Monoschicht bedeckt ist, die eine Carboxylatgruppe, ein Metallchelat, wie etwa Nitrilotriessigsäure, Biotin, N-Hydroxysuccinimid, Glutathion, Streptavidin oder Fragment davon beinhaltet, die nur ein Biotin und ein Oligonukleotid bindet.

24. Kit gemäß Anspruch 23, wobei das Nanopartikeln eine Carboxylatgruppe, ein Metallchelat, wie etwa Nitrilotriessigsäure, Biotin, N-Hydroxysuccinimid, Glutathion, Streptavidin oder Fragment davon trägt, die nur ein Biotin bindet, um so das Protein oder Peptid am Nanopartikel zu binden.

25. Kit gemäß Anspruch 23 oder Anspruch 24, weiterhin beinhaltend eine Vielzahl von Oberflächen, von denen jede ein Protein oder Peptid trägt oder angepasst ist, ein Protein oder Peptid zu tragen;
wobei jede Oberfläche das Protein oder Peptid speziell bindet oder angepasst ist, das Protein oder Peptid speziell zu binden.

26. Kit, beinhaltend eine Vielzahl von Goldnanopartikeln,
wobei die Oberfläche von jedem Nanopartikel vollständig mit einer selbst organisierten Monoschicht bedeckt ist, die Thiole, terminiert mit einem Bindungspartner eines Affinitäts-Tags, und ein Oligonukleotid beinhaltet.

27. Kit gemäß Anspruch 26, wobei das Affinitäts-Tag ein Metallbindungs-Tag ist und der Bindungspartner des Affinitäts-Tags ein Stoff ist, in einem Chelat terminierend, ein Metall koordinierend, oder wobei das Affinitäts-Tag GST ist und der Bindungspartner des Affinitäts-Tags Glutathion ist, oder wobei das Affinitäts-Tag Streptavidin ist und der Bindungspartner des Affinitäts-Tags Biotin ist.

28. Kit gemäß Anspruch 27, wobei das Affinitäts-Tag ein Histidin-Tag und der Bindungspartner des Affinitäts-Tags NTA-Nickel ist.

29. Kit gemäß einem der Ansprüche 23 bis 28, wobei das Oligonukleotid das Protein oder Peptid codiert.

30. Kit gemäß einem der Ansprüche 23 bis 29, wobei die Nanopartikel jeweils ein Protein oder Peptid tragen.

31. Kit gemäß Anspruch 30, wobei das Protein oder Peptid ein Antikörper oder Antigen ist.

## Revendications

1. Procédé de dépistage simultané des interactions entre des espèces chimiques ou biologiques, consistant à :
fournir une première espèce chimique ou biologique, immobilisée par rapport à une première surface, et un premier identificateur d'oligonucléotides indépendamment associé à la première surface ;
fournir une deuxième espèce chimique ou biologique, immobilisée par rapport à une deuxième surface ;
permettre à la première espèce de participer à une interaction chimique ou biologique avec la deuxième espèce ;
déterminer la participation de la première espèce et de la deuxième espèce dans l'interaction ; et
déterminer l'identité du premier identificateur d'oligonucléotides, identifiant ainsi la première espèce ;
où la première surface est la surface d'une nanoparticule.

2. Procédé selon la revendication 1, où la nanoparticule est une particule colloïdale.

3. Procédé selon la revendication 2, où la nanoparticule est une particule colloïdale d'or.

4. Procédé selon l'une quelconque des revendications précédentes, où la deuxième surface est la surface d'une particule recrutable, d'une bille magnétique, d'une particule colloïdale ou d'une puce.

5. Procédé selon la revendication 4, où la deuxième surface est la surface d'une bille.

6. Procédé selon l'une quelconque des revendications précédentes, où le premier identificateur d'oligonucléotides code la première espèce.

7. Procédé selon la revendication 6, où la première espèce est une protéine.

8. Procédé selon l'une quelconque des revendications précédentes, où la nanoparticule est une particule colloïdale, et la première espèce et le premier identificateur d'oligonucléotides sont immobilisés par rapport à la surface de la particule colloïdale par l'intermédiaire d'une monocouche auto-assemblée.

9. Procédé selon la revendication 8, où la première espèce est fixée à la surface de la particule colloïdale par une liaison de type étiquette d'attache métallique/métal/chélate.

10. Procédé selon la revendication 8 ou la revendication 9, où le premier identificateur d'oligonucléotides comprend une espèce d'oligonucléotides comprenant une partie liant qui est le complément d'un liant d'oligonucléotides à la surface de la particule colloïdale, et le premier identificateur d'oligonucléotides est fixé à la surface de la particule colloïdale par l'intermédiaire du liant d'oligonucléotides.

11. Procédé selon l'une quelconque des revendications précédentes, où le premier identificateur d'oligonucléotides est immobilisé à la première surface via une protéine de liaison d'acides nucléiques, de préférence par l'intermédiaire d'une protéine qui se lie à l'ADN.

12. Procédé selon l'une quelconque des revendications 1 à 10, où le premier identificateur d'oligonucléotides est modifié pour faciliter l'attachement à la première surface par l'intermédiaire d'une protéine de reconnaissance.

13. Procédé selon l'une quelconque des revendications 1 à 10, où le premier identificateur d'oligonucléotides est biotinylé pour faciliter l'attachement à la première surface par l'intermédiaire de streptavidine.

14. Procédé selon l'une quelconque des revendications précédentes, où le premier identificateur d'oligonucléotides comprend un ADN plasmidique, un vecteur d'expression de protéines ou un ADN linéaire.

15. Procédé selon l'une quelconque des revendications précédentes, où le premier identificateur d'oligonucléotides comprend un modèle d'expression de protéines et/ou un produit d'une réaction en chaîne de la polymérase.

16. Procédé selon l'une quelconque des revendications précédentes, où une entité de signalisation est immobilisée par rapport à au moins l'un du premier identificateur d'oligonucléotides et de la première espèce.

17. Procédé selon l'une quelconque des revendications 1 à 16, où le premier identificateur d'oligonucléotides est modifié pour inclure une entité de signalisation.

18. Procédé selon la revendication 17, où le premier identificateur d'oligonucléotides est généré par PCR en utilisant des amorces modifiées par des entités de signalisation.

19. Procédé selon l'une quelconque des revendications 16 à 18, où la séquence du premier identificateur d'oligonucléotides est déterminée par séquençage fluorescent.

20. Procédé selon l'une quelconque des revendications précédentes, où un deuxième identificateur d'oligonucléotides est associé de façon indépendante à la deuxième surface, comprenant la détermination de la séquence du premier identificateur d'oligonucléotides et du deuxième identificateur d'oligonucléotides.

21. Procédé selon la revendication 20, consistant à :
permettre à la première espèce et à la deuxième espèce de participer à l'interaction, en immobilisant ainsi la première particule colloïdale et la deuxième particule colloïdale l'une par rapport à l'autre et en approchant le premier identificateur d'oligonucléotides du deuxième identificateur d'oligonucléotides ;
exposer le premier identificateur d'oligonucléotides et le deuxième identificateur d'oligonucléotides à un identificateur d'hybridation d'interaction, qui est complémentaire de la combinaison du premier identificateur d'oligonucléotides et du deuxième identificateur d'oligonucléotides, et permettant à l'identificateur d'hybridation d'interaction, de se lier au premier identificateur d'oligonucléotides et au deuxième identificateurs d'oligonucléotides ; et
identifier l'identificateur d'hybridation d'interaction, en identifiant ainsi le premier identificateur d'oligonucléotides et le deuxième identificateur d'oligonucléotides, et ainsi identifiant l'interaction.

22. Procédé selon la revendication 21, consistant, avant l'étape d'identification, à désactiver tout oligonucléotide non-hybridé.

23. Kit comprenant une pluralité de nanoparticules d'or,
où la surface de chaque nanoparticule est complètement recouverte d'une monocouche auto-assemblée qui incorpore un groupe carboxylate, un chélate de métal comme l'acide nitrilotriacétique, la biotine, le n-hydroxy succinimide, le glutathion, la streptavidine ou un fragment qui lie une seule biotine
et un oligonucléotide.

24. Kit selon la revendication 23, où la nanoparticule porte un groupe carboxylate, un chélate de métal comme l'acide nitrilotriacétique, la biotine, le n-hydroxy succinimide, le glutathion, la streptavidine ou un fragment qui lie une seule biotine
pour lier la protéine ou le peptide à la nanoparticule.

25. Kit selon la revendication 23 ou la revendication 24, comprenant en outre une pluralité de surfaces qui chacune porte ou est adaptée pour porter une protéine ou un peptide ;
où chaque surface se lie spécifiquement ou est adaptée pour se lier spécifiquement à la protéine ou au peptide.

26. Kit comprenant une pluralité de nanoparticules d'or,
où la surface de chaque nanoparticule est complètement recouverte par une monocouche auto-assemblée qui incorpore des thiols terminés par un partenaire de liaison d'une étiquette d'affinité, et un oligonucléotide.

27. Kit selon la revendication 26, où l'étiquette d'affinité est une étiquette de liaison de métal et le partenaire de liaison de l'étiquette d'affinité est une espèce se terminant par un chélate de coordination d'un métal, ou bien dans lequel l'étiquette d'affinité est GST et le partenaire de liaison de l'étiquette d'affinité est le glutathion, ou bien dans lequel l'étiquette d'affinité est la streptavidine et le partenaire de liaison de l'étiquette d'affinité est la biotine.

28. Kit selon la revendication 27, où l'étiquette d'affinité est une étiquette d'histidine et le partenaire de liaison de l'étiquette d'affinité est le NTA-nickel.

29. Kit selon l'une quelconque des revendications 23 à 28, où l'oligonucléotide code la protéine ou le peptide.

30. Kit selon l'une quelconque des revendications 23 à 29, où les nanoparticules portent chacune une protéine ou un peptide.

31. Kit selon la revendication 30, où la protéine ou le peptide est un anticorps ou un antigène.
